(19)

**Europäisches Patentamt**
**European Patent Office**
**Office européen des brevets**

(11) **EP 4 734 046 A1**

(12) # EUROPEAN PATENT APPLICATION

(43) Date of publication:
  29.04.2026  Bulletin 2026/18

(21) Application number: 25211092.9

(22) Date of filing: **24.10.2025**

(51) International Patent Classification (IPC):
  ***G06T 5/60*** *(2024.01)*   ***G06T 5/70*** *(2024.01)*
  ***G06T 12/10*** *(2026.01)*

(52) Cooperative Patent Classification (CPC):
  **G06T 5/70; G06T 5/60; G06T 12/10;**
  G06T 2207/10081; G06T 2207/10104;
  G06T 2207/20081; G06T 2207/20084

(84) Designated Contracting States:
  **AL AT BE BG CH CY CZ DE DK EE ES FI FR GB**
  **GR HR HU IE IS IT LI LT LU LV MC ME MK MT NL**
  **NO PL PT RO RS SE SI SK SM TR**
  Designated Extension States:
  **BA**
  Designated Validation States:
  **GE KH LA MA MD TN**

(30) Priority: 24.10.2024  CN 202411488889
      13.12.2024  CN 202411850653

(71) Applicant: **Shanghai United Imaging Healthcare**
  **Co., Ltd.**
  **Shanghai 201807 (CN)**

(72) Inventors:
  • XU, Hancong
    **Shanghai, 201807 (CN)**
  • XIE, Huifang
    **Shanghai, 201807 (CN)**
  • LV, Yang
    **Shanghai, 201807 (CN)**
  • DONG, Yun
    **Shanghai, 201807 (CN)**

(74) Representative: **Balder IP Law, S.L.**
  **Paseo de la Castellana 93**
  **5ª planta**
  **28046 Madrid (ES)**

(54) **EMISSION TOMOGRAPHY IMAGE PROCESSING METHOD AND APPARATUS, COMPUTER DEVICE, AND STORAGE MEDIUM**

(57)   An emission tomography image processing method includes: acquiring local medical images of a target object, where different local medical images correspond to different spatial positions or different tissue regions; predicting noise level information corresponding to the local medical images; and performing, based on the noise level information corresponding to the local medical images, image denoising on the local medical images to obtain a target medical image, where at least two of the local medical images correspond to different noise level information.

**EP 4 734 046 A1**

**Description**

**TECHNICAL FIELD**

The present disclosure relates to the field of image processing technologies, and in particular, to an emission tomography image processing method and an apparatus, a computer device, and a storage medium.

**BACKGROUND**

Medical images are affected by various factors during the process of acquisition, transmission, and processing, which may cause noise in the medical images and reduce the quality of the medical images.

In order to avoid the impact of noise on the quality of medical images, in related technologies, a noise perception network is mainly used to predict the noise level of the entire image and perform adaptive denoising based on the predicted results.

However, based on methods in the related art, all the noise in the image from a holistic perspective cannot be obtained. Therefore, the noise in the image cannot be removed more comprehensively through the denoising, resulting in a poor denoising effect.

**SUMMARY**

An emission tomography image processing method and an apparatus, a computer device, and a storage medium are provided in the present disclosure.

An emission tomography image processing method is provided in the present disclosure. The method includes:

acquiring local medical images of a target object, where different local medical images correspond to different spatial positions or different tissue regions;

predicting noise level information corresponding to the local medical images; and

performing, based on the noise level information corresponding to the local medical images, image denoising on the local medical images to obtain a target medical image, where at least two of the local medical images correspond to different noise level information.

In some embodiments, the different spatial positions include different axial slices.

In some embodiments, acquiring the local medical images of the target object includes acquiring local medical images of the target object corresponding to different axial slices, and predicting the noise level information corresponding to the local medical images includes:

inputting the local medical images corresponding to the axial slices into an axial slice noise prediction model, and predicting a noise level of the local medical images corresponding to the different axial slices using the axial slice noise prediction model to obtain noise level information corresponding to the different axial slices.

In some embodiments, acquiring the local medical images of the target object includes acquiring local medical images of the target object corresponding to different tissue regions, and predicting the noise level information corresponding to the local medical images includes:

inputting the local medical images corresponding to the tissue regions into a regional noise prediction model, and predicting a noise level of the local medical images corresponding to different tissue regions using the regional noise prediction model to obtain noise level information corresponding to the different tissue regions.

In some embodiments, performing, based on the noise level information corresponding to the local medical images, image denoising on the local medical image to obtain the target medical image includes:

performing, based on the noise level information corresponding to the local medical images, image denoising on the local medical image using a predetermined denoising model to obtain the target medical image.

In some embodiments, the emission tomography image processing method further includes training the denoising model. Training the denoising model includes:

acquiring sample medical images and sample local medical images of a sample object, the sample local medical images including at least one of sample local medical images corresponding to a plurality of axial slices and sample local medical images corresponding to a plurality of tissue regions;

predicting a noise level of the sample local medical images using a noise prediction model to obtain noise level information, the noise prediction model including at least one of an axial slice noise prediction model and a regional noise prediction model;

fusing the noise level information with the sample medical images to obtain fused sample medical images; and

training an initial denoising model using the fused sample medical images until an output result of the initial denoising model meets a predetermined condition, to obtain the denoising model.

In some embodiments, the different axial slices have different relaxation factors.

In some embodiments, acquiring the local medical images of the target object corresponding to the different axial slices includes:

in a step S1, updating an initial PET image of each axial slice based on PET projection data to obtain an updated PET image of the axial slice;

in a step S2, obtaining a relaxation factor of each axial slice;

in a step S3, obtaining, for each axial slice, a first weight and a second weight of the axial slice based on the relaxation factor of the axial slice, and performing a weighted summation on the updated PET image of the axial slice and the initial PET image of the axial slice based on the first weight and the second weight to obtain a weighted PET image of the axial slice;

in a step S4, obtaining, on a condition that the weighted PET image of each axial slice meets an iteration end condition, the local medical images corresponding to the axial slices based on weighted PET images of the axial slices; and

in a step S5, determining, on a condition that the weighted PET image of each axial slice does not meet the iteration end condition, the weighted PET image of each axial slice as the initial PET image of each axial slice, and returning to execute steps S1 to S3.

In some embodiments, obtaining the relaxation factors of the axial slices includes:

obtaining, for each axial slice, a slice number difference between the axial slice and a central axial slice; and

determining, on a condition that the slice number difference is greater than a slice number difference threshold, the relaxation factor of the axial slice based on the slice number difference and a predetermined mapping relationship, the relaxation factor of the axial slice being smaller than a relaxation factor of the central axial slice.

In some embodiments, the method further includes:

determining, on a condition that the slice number difference is less than or equal to the slice number difference threshold, the relaxation factor of the central axial slice as the relaxation factor of the axial slice.

In some embodiments, obtaining the relaxation factors of the axial slices includes:

obtaining, for each voxel on the axial slice, a detection probability of each line of response (LOR) for the voxel;

obtaining an attenuation coefficient of each LOR; and

obtaining, for each axial slice, the relaxation factor of the axial slice based on the detection probability of each LOR of each voxel on the axial slice and the attenuation coefficient of each LOR of each voxel.

In some embodiments, obtaining, for each axial slice, the relaxation factor of the axial slice based on the detection probability of each LOR of each voxel on the axial slice and the attenuation coefficient of each LOR of each voxel includes:

performing, for each voxel on the axial slice, a weighted summation on the detection probability of each LOR of the voxel and the attenuation coefficient of each LOR to obtain a detection probability weighted value of the voxel; and

obtaining the relaxation factor of the axial slice based on the detection probability weighted value of each voxel on the axial slice.

In some embodiments, obtaining the first weight and the second weight of the axial slice based on the relaxation factor of the axial slice includes:

obtaining the first weight of the axial slice based on the relaxation factor of the axial slice; and

obtaining the second weight of the axial slice based on a difference between a predetermined value and the relaxation factor of the axial slice.

In some embodiments, updating the initial PET image of each axial slice based on the PET projection data to obtain the updated PET image of each axial slice includes:

obtaining first PET projection prediction data based on an initial PET image of each axial slice; and

updating the initial PET image of each axial slice based on differences between the first PET projection prediction data and the PET projection data to obtain the updated PET image of each axial slice.

In some embodiments, the method further includes:

obtaining second PET projection prediction data based on the weighted PET image of each axial slice; and

determining whether the iteration end condition is met based on differences between the second PET projection prediction data and the PET projection data.

In some embodiments,the iteration end condition includes that a mean square error or a sum of absolute differences between the second PET projection prediction data and the PET projection data is less than a difference threshold.

In some embodiments,the noise level information is scaled or smoothed before fusing with the sample medical images.

An emission tomography image processing apparatus is provided in the present disclosure, including:

an acquisition module configured to acquire local medical images of a target object, wherein different local medical images correspond to different spatial positions or different tissue regions;

a prediction module configured to predict noise level information corresponding to the local medical images; and

a denoising module configured to perform, based on the noise level information corresponding to the local medical images, image denoising on the local medical images to obtain a target medical image, wherein at least two of the local medical images correspond to different noise level information.

A computer device is provided in the present disclosure, including a memory and a processor. The memory stores a computer program. The processor, when executing the computer program, implements the steps of the above-mentioned method.

A non-transitory computer-readable storage medium having a computer program stored thereon is provided in the present disclosure. When the computer program is executed by a processor, the steps of the above-mentioned method are implemented.

## BRIEF DESCRIPTION OF THE DRAWINGS

In order to more clearly illustrate the technical solutions in the embodiments of the present disclosure or related technologies, the following briefly introduces the drawings required for use in the embodiments of the present disclosure or related technical descriptions. Apparently, the drawings described below are only some embodiments of the present disclosure. For those skilled in the art, other related drawings can be obtained based on these drawings without paying any creative work.

FIG. 1 is a diagram showing an application environment of an emission tomography image processing method in some embodiments.

FIG. 2 is a schematic flowchart of emission tomography image processing method in some embodiments.

FIG. 3 is a local medical image of a plurality of axial slices in some embodiments.

FIG. 4 is a schematic flowchart of an emission tomography image processing method in some embodiments.

FIG. 5 is a schematic flowchart of an emission tomography image processing method in some embodiments.

FIG. 6 is schematic flowchart of an overall process of adaptive denoising using a noise-aware network to assist a denoising network.

FIG. 7 is a schematic flowchart of an emission tomography image processing method in some embodiments.

FIG. 8 is a block diagram of a structure of an emission tomography image processing apparatus in some embodiments.

FIG. 9 is a schematic diagram of a detector structure used in a PET system in some embodiments.

FIG. 10 is a schematic flowchart of a PET image reconstruction method in some embodiments.

FIG. 11 (a) is a schematic diagram showing a change of a relaxation factor with the axial slice in some embodiments.

FIG. 11 (b) is a schematic diagram showing a change of a relaxation factor with axial slice in other embodiments.

FIG. 12 is a schematic diagram showing a change of a relaxation factor with axial slices in yet other embodiments.

FIG. 13 is a block diagram of a structure of a PET image reconstruction apparatus in some embodiments.

## DETAILED DESCRIPTION OF THE EMBODIMENTS

The following will be combined with the accompanying drawings in the embodiments of the present disclosure to clearly and completely describe the technical solutions in the embodiments of the present disclosure. Apparently, the embodiments described are only part of the embodiments of the present disclosure, not all of the embodiments. Based on the embodiments in the present disclosure, all other embodiments obtained by those skilled in the art without making creative efforts are within the scope of protection of the present disclosure.

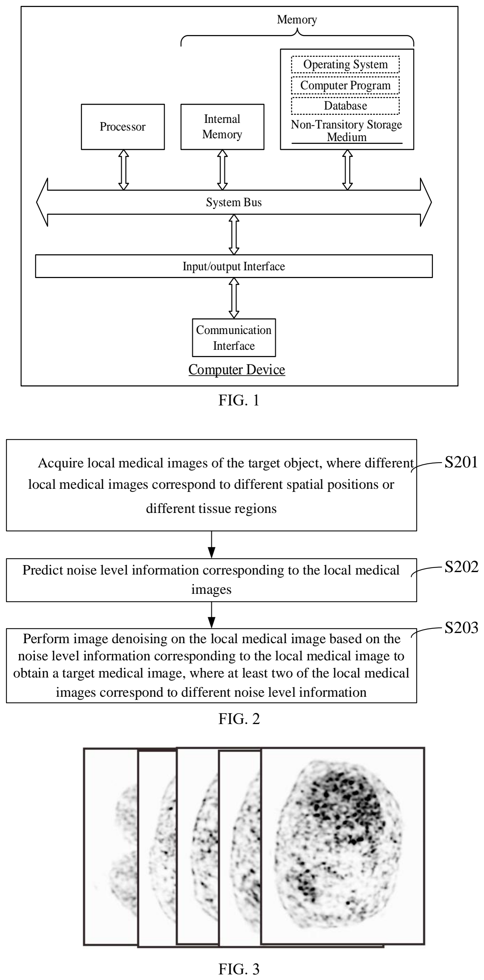

The emission tomography image processing method provided in the embodiments of the present disclosure can be applied in an application environment shown in FIG. 1. The computer device in the application environment can be a server, a personal computer, a laptop computer, a smart phone, a tablet computer, a smart mobile phone, etc. The computer device may include a processor, a memory and a network interface connected via a system bus or wirelessly. The processor of the computer device is configured to provide computing and control capabilities. The memory of the computer device may include a non-transitory storage medium and an internal memory. The non-transitory storage medium stores an operating system, a computer program and a database. The internal memory provides an environment for operations of an operating system and the computer program in the non-transitory storage medium. The database of the computer device is configured to store data during the emission tomography image processing. The network interface of the computer device is configured to communicate with an external terminal via a network connection, and the computer program is executed by the processor to implement an emission tomography image processing method.

In an exemplary embodiment, as shown in FIG. 2, an emission tomography image processing method is provided. Taking the method applied to the computer device in FIG. 1 as an example, the method includes the following steps S201 to S203.

In a step S201, local medical images of a target object is acquired, where different local medical images correspond to different spatial positions or different tissue regions.

In which, an initial medical image of the target object can be obtained. The initial medical image can be a two-dimensional medical image or a three-dimensional medical image, and the initial medical image contains different types of noise. On a condition that the initial medical image is a two-dimensional medical image, the initial medical image can be segmented based on tissue regions to obtain local medical images. Then, the obtained local medical images are also two-dimensional medical images. On a condition that the initial medical image is a three-dimensional medical image, the initial medical image can be segmented based on tissue regions to obtain local medical images. The obtained local medical images are three-dimensional local medical images corresponding to different tissue regions, and image noise levels of different tissue regions are different. The different spatial positions include different axial slices. Alternatively, the initial medical image, which is a three-dimensional image stored in accordance with the DICOM (Digital Imaging and Communications in Medicine) standard, e.g., stored with the (x, y, z) storage standard, can be sliced along different dimensions to obtain local medical images corresponding to different two-dimensional slices. For example, based on a axial slice dimension which can be determined according to spatial aggregation position information of a PET detector or any geometric angle, local medical images corresponding to different axial slices can be obtained. Based on the tissue region dimension which can be determined according to anatomical information of the target object, such as liver region, spleen region, kidney region, etc., local medical images corresponding to different tissue regions can be obtained. Moreover, the local medical images corresponding to different axial slices can be further segmented based on different tissue regions. It should be noted that the tissue regions are not limited to two-dimensional, they can also be three-dimensional. In some embodiments, the obtained local medical images are two-dimensional local medical images corresponding to different slices, and the image noise levels of different slices are different.

Traditional image quality assessments often focus on the ROI (region of interest), ultimately outputting a single overall quality score. However, noise differs fundamentally from conventional image features in that its distribution is not confined to the ROI but encompasses every pixel in an entire image. Therefore, relying solely on ROI quality grading cannot accurately characterize and predict the noise level across the entire image.

Taking PET (positron emission tomography) images as an example, in the past, the noise levels of specific areas, such as the liver and blood pool, were often used instead of the overall image noise assessment. This approach was essentially a compromise to reduce computational complexity and simplify the operational process. However, in actual disclosures, the local noise characteristics of the liver and blood pool are not sufficient to represent the true noise levels of all tissue regions in the body, and the assessment results have obvious limitations. In some examples, local medical images are not limited to corresponding to different axial slices and different tissue regions, but can also be obtained by analyzing different pixels in the initial medical image. Specifically, a noise level estimation network can perform image quality assessment for each pixel in the image and assign a corresponding score, to obtain noise levels for different pixels in the image.

The initial medical image of the target object can be a positron emission tomography (PET) or single-photon emission computed tomography (SPECT) image. Since a detector structure in a PET or SPECT device is annular and the sensitivity varies at different detector locations, the noise level at different reconstructed image locations (for example, different axial slices) varies. This requires learning the noise level for each spatial location and performing adaptive denoising. Furthermore, due to differences in attenuation and tissue properties among different organs or tissues in the image, the noise level also varies, necessitating adaptive denoising.

In some embodiments, the local medical images correspond to at least one of image classification dimensions, and the image classification dimensions include an axial slice dimension and a tissue region dimension. On a condition that the initial medical image has been segmented based on at least one of image classification dimensions, and a plurality of local medical images obtained by the segmenting are stored in a Picture Archiving and Communication System (PACS), the computer device can obtain the local medical images of the target object from the PACS based on identification information of the initial medical image.

Optionally, after receiving an image denoising instruction for the target object, the computer device may also segment the initial medical image in at least one of image classification dimensions to obtain the local medical images of the target object. The method for obtaining the local medical images of the target object is not limited in the embodiments of the present disclosure.

In a step S202, noise level information corresponding to the local medical images is predicted.

The noise level information is configured to evaluate a noise level of each local medical image. The noise level information can include a quantified noise level value. For example, the quantified noise level value ranges from 0 to 1. On a condition that the noise level of a local medical image is close to 1, it indicates that the noise level of the local medical image is relatively high. On a condition that the noise level of a local medical image is close to 0, it indicates that the noise level of the local medical image is relatively low.

In some embodiments, the computer device can input each local medical image into a predetermined noise prediction model, predict a noise level of each local medical image through the noise prediction model, and obtain a quantified noise level value corresponding to each local medical image. Alternatively, for any local medical image, the computer device can obtain pixel values of a plurality of pixels of the local medical image and add the pixel values of the pixels. Then, based on calculation rules of the quantified noise level value, a sum of the pixel values of each local medical image is analyzed to determine the quantified noise level value corresponding to each local medical image. Alternatively, quantified noise level values for pixels in the image may also be obtained, and the quantified noise level values (e.g., a multi-value array between 0 and 1) may be filled into an image spatially aligned with the original image, thereby obtaining the quantified noise level value corresponding to each local medical image. A method for predicting the quantified noise level value corresponding to the local medical images is not limited in the embodiments of the present disclosure.

In a step S203, image denoising is performed on the local medical image based on the noise level information corresponding to the local medical images to obtain a target medical image, where at least two of the local medical images correspond to different noise level information.

In some embodiments, for images with a higher noise level, a higher degree of denoising is required, and for images with a lower noise level, a lower degree of denoising is required. That is, there is a positive correlation between the quantified noise level value of the local medical image and a degree of denoising for the local medical image. For example, on a condition that a noise level of a local medical image is close to 1, a higher degree of denoising is required for the local medical image. On a condition that a noise level of a local medical image is close to 0, a lower degree of denoising is required for the local medical image, or no denoising is required for the local medical image.

Therefore, for any local medical image, after obtaining the quantified noise level value corresponding to the local medical image, the computer device can determine a quantified noise reduction degree corresponding to the local medical image based on a mapping relationship between the quantified noise level value and the quantified noise reduction degree. Based on the quantified noise reduction degree corresponding to the local medical image, the computer device performs denoising on the local medical image to obtain a target medical image.

Optionally, the computer device can further input the local medical images and corresponding quantified noise level values into a predetermined denoising model, and perform denoising on each local medical image based on the quantified noise level value corresponding to each local medical image through the predetermined denoising model to obtain a target medical image.

In the above-mentioned emission tomography image processing method, local medical images of a target object is acquired. The local medical image corresponds to at least one of image classification dimensions. The image classification dimensions include an axial slice dimension and a tissue region dimension. A quantified noise level value corresponding to the local medical image is predicted. Based on the quantified noise level value corresponding to the local medical image, image denoising is performed on the local medical image to obtain a target medical image. This method is capable of acquiring a local medical image based on at least one of image classification dimensions and predicting the quantified noise level value of the local medical image. Based on the prediction result, the local medical image is then adaptively denoised, i.e., denoising is performed from a local perspective, thereby improving a denoising effect of the obtained target medical image.

The at least one of image classification dimensions may be an axial slice dimension and a tissue region dimension.

In some embodiments, the at least one of image classification dimensions includes the axial slice dimension. Acquiring the local medical images of the target object includes acquiring local medical images of the target object corresponding to different axial slices.

The local medical images corresponding to different axial slices refer to two-dimensional medical image slices obtained by segmenting the target object (such as a human body, organs, etc.) in a craniocaudal direction as the axial dimension through planes parallel to the axial direction, which are used to present local structures and details of the target object at a specific height position.

FIG. 3 shows a local medical image of a plurality of axial slices, where each axial slice refers to a local medical image at a fixed height.

In some embodiments, after obtaining a target medical image, the computer device may segment the target medical image along an axial direction based on a predetermined height requirement to obtain local medical images corresponding to different axial slices. It should be noted that when at least one of image classification dimensions is an axial slice dimension, the initial medical image is a three-dimensional medical image, and the corresponding local medical images corresponding to different axial slices are all two-dimensional local medical images.

In the above-mentioned emission tomography image processing method, local medical images corresponding to different axial slices of the target object are obtained. In this method, the division dimension of the target medical image is limited so that a segmenting process is performed along the axial dimension, thereby accurately obtaining local medical images corresponding to different axial slices.

On a condition that at least one of image classification dimensions includes an axial slice dimension, a noise prediction process is to predict local medical images corresponding to different axial slices. In some embodiments, predicting the quantified noise level value corresponding to the local medical images includes:

inputting the local medical images corresponding to the axial slices into an axial slice noise prediction model, and predicting a noise level of the local medical images corresponding to the different axial slices using the axial slice noise prediction model to obtain noise level informations corresponding to the different axial slices.

The axial slice prediction model is configured to predict a noise level in local medical images corresponding to different axial slices. It can be a deep neural network (DNN), a recurrent neural network (RNN), or a convolutional neural network (CNN). CNNs can be ResNet, CPN, SimpleBaseline, Posefix, HRNet, or a deep learning-based human pose estimation algorithm (HigherHRNet). An axial slice prediction model can include a single network or a combination of a plurality of networks.

During a training process of the axial slice prediction model, sample local medical images corresponding to different axial slices and corresponding true quantified noise level values are obtained. The sample local medical images corresponding to different axial slices are input into an initial axial slice prediction model, and the initial axial slice prediction model is configured to predict the noise level of each axial slice to obtain a predicted quantified noise level value. Based on a difference between the predicted quantified noise level value and a true quantified noise level value, parameters of the initial axial slice prediction model are adjusted until the difference between the predicted quantified noise level value output by the initial axial slice prediction model and the true quantified noise level value is less than a predetermined threshold. The initial axial slice prediction model training is completed, and the axial slice prediction model is obtained.

In some embodiments, after acquiring local medical images corresponding to different axial slices, the computer device may use the local medical images corresponding to the different axial slices as input signals and input them into an axial slice noise prediction model. The axial slice noise prediction model may first extract feature information from the local medical images corresponding to each axial slice and perform noise prediction based on extracted feature information to obtain quantified noise level values corresponding to the local medical images corresponding to each axial slice.

In the above-mentioned emission tomography image processing method, the local medical images corresponding to the axial slices are input into an axial slice noise prediction model. The axial slice noise prediction model is configured to predict the noise level of the local medical images corresponding to different axial slices, to obtain the quantified noise level values corresponding to the axial slices. This method utilizes the axial slice noise prediction model to specifically analyze local medical images corresponding to different axial slices, accurately predicting the noise level corresponding to each local medical image of each axial slice, and thus achieving a more accurate quantified noise level value.

In some embodiments, at least one of image classification dimensions includes the tissue region dimension, and acquiring the local medical images of the target object includes acquiring local medical images of the target object corresponding to different tissue regions.

The local medical images of the target object corresponding to different tissue regions are acquired, and the initial medical image of the target object can be based on tissue regions without being limited to a specific direction, such as a horizontal dimension which refers to the dimension of a direction in which a human body lies flat.

In some embodiments, after acquiring the target medical image, the computer device may input the target medical image into a coarse segmentation model, which can be a deep learning model, an algorithm model, or open-source software such as TotalSegmentator, Moose, etc., capable of segmenting tissues to obtain VOI (Volume of Interest) or ROI (Region of Interest) of tissues. The computer device may use the coarse segmentation model to segment the initial medical image based on the tissue regions to obtain local medical images corresponding to the tissue regions.

In the aforementioned emission tomography image processing method, the initial medical image is segmented based on the tissue regions to obtain the local medical images corresponding to the plurality of tissue regions. By limiting segmentation dimensions of the target medical image, this method ensures that a segmentation process proceeds along the tissue regions, enabling accurate acquisition of the local medical images corresponding to the plurality of tissue regions.

On a condition that at least one of image classification dimensions includes the tissue region dimension, the noise prediction process is to predict local medical images corresponding to different tissue regions. In some embodiments, predicting the noise level information corresponding to the local medical images includes:

inputting the local medical images corresponding to the tissue regions into a regional noise prediction model, and predicting a noise level of the local medical images corresponding to different tissue regions using the regional noise prediction model to obtain noise level information corresponding to the different tissue regions.

The regional noise prediction model is configured to predict a noise level in the local medical images corresponding to different tissue regions. The regional noise prediction model and the axial slice prediction model can have the same network structure. For example, the regional noise prediction model can be a DNN, RNN, or CNN model, etc.

During the training process of the regional noise prediction model, the sample local medical images corresponding to the plurality of tissue regions and the corresponding true quantified noise level values are obtained. The sample local medical images corresponding to the plurality of tissue regions are input into the initial regional noise prediction model, and the initial regional noise prediction model is configured to predict the noise level of the sample local medical images corresponding to different tissue regions to obtain a predicted quantified noise level value. Based on the difference between the predicted quantified noise level value and the true quantified noise level value, the parameters of the initial regional noise prediction model are adjusted until the difference between the predicted quantified noise level value output by the initial regional noise prediction model and the true quantified noise level value is less than a predetermined threshold. The initial regional noise prediction model training is completed, and the regional noise prediction model is obtained.

In some embodiments, after acquiring local medical images corresponding to the plurality of tissue regions, the computer device may use the local medical images corresponding to the plurality of tissue regions as input signals to a regional noise prediction model. The regional noise prediction model may first extract feature information from the local medical images corresponding to different tissue regions and perform noise prediction based on the extracted feature information to obtain a quantified noise level value corresponding to the local medical images corresponding to different tissue regions.

In the aforementioned emission tomography image processing method, the local medical images corresponding to the tissue regions are input into a regional noise prediction model. The regional noise prediction model is configured to predict the noise level of the local medical images corresponding to different tissue regions, resulting in noise level information corresponding to each tissue region. This method utilizes the regional noise prediction model to specifically analyze the local medical images corresponding to a plurality of tissue regions, accurately predicting the noise level corresponding to each local medical image of each tissue region, and thus achieving a more accurate noise level information.

In some embodiments, performing image denoising on the local medical image based on the noise level information corresponding to the local medical images to obtain a target medical image includes:

based on the noise level information corresponding to the local medical images, performing image denoising on the local medical image using a predetermined denoising model to obtain a target medical image.

In some embodiments, after obtaining the noise level information of each local medical image, each local medical image and the corresponding noise level information can be input into a predetermined denoising model, and each local medical image can be denoised by the denoising model to obtain the target medical image.

Specifically, the local medical images may be local medical images corresponding to different axial slices. During the denoising process, the denoising model performs denoising on any local medical image of an axial slice using the quantified noise level value corresponding to the local medical image. A denoised local medical images corresponding to the different axial slices are then spliced together to obtain a target medical image.

Optionally, the local medical images may be local medical images corresponding to different tissue regions.

During a noise reduction process, the denoising model performs denoising on any local medical image of the tissue region using the quantified noise level corresponding to the local medical image. A denoised local medical images corresponding to the different tissue regions are then spliced together to obtain a target medical image.

Optionally, the local medical images may be local medical images corresponding to different axial slices or local medical images corresponding to different tissue regions. During the noise reduction process, the denoising model simultaneously considers the quantified noise level value of each axial slice and the quantified noise level value of each tissue region, thereby achieving a better denoising effect.

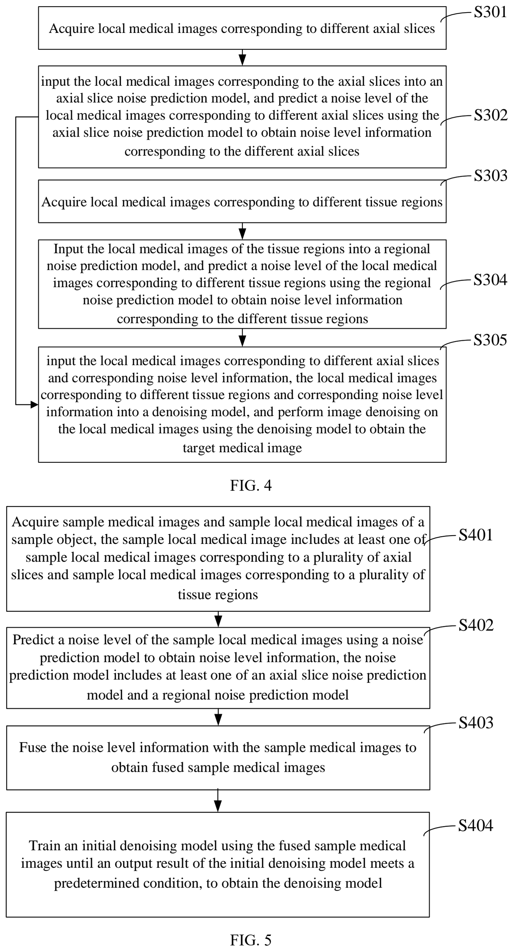

In the above-described emission tomography image processing method, based on the noise level information corresponding to the local medical images, a predetermined denoising model is used to perform image denoising on the local medical images to obtain a target medical image. Based on the noise level information corresponding to the local medical images, this method utilizes the denoising model to specifically reduce noise in each local medical image of the target object, thereby improving the denoising effect of the obtained target medical image. In some embodiments, as shown in FIG. 4, the above-described emission tomography image processing method includes the following steps S301 to S305.

In a step S301, local medical images corresponding to different axial slices are acquired.

In a step S302, the local medical images corresponding to the axial slices are input into an axial slice noise prediction model, and a noise level of the local medical images corresponding to different axial slices are predicted using the axial slice noise prediction model to obtain noise level information corresponding to the different axial slices.

In a step S303, local medical images corresponding to different tissue regions are acquired.

In a step S304, the local medical images corresponding to the tissue regions are input into a regional noise prediction model, and a noise level of the local medical images corresponding to different tissue regions are predicted using the regional noise prediction model to obtain noise level information corresponding to the different tissue regions.

In a step S305, the local medical images corresponding to different axial slices and corresponding noise level information, the local medical images corresponding to different tissue regions and corresponding noise level information are input into a denoising model, and image denoising is performed on the local medical images using the denoising model to obtain the target medical image.

As shown in FIG. 5, the emission tomography image processing method further includes training the denoising model. Training the denoising model includes steps S401 to S404.

In a step S401, sample medical images and sample local medical images of a sample object are acquired, the sample local medical image includes at least one of sample local medical images corresponding to a plurality of axial slices and sample local medical images corresponding to a plurality of tissue regions.

In some embodiments, the sample medical images of the sample object and at least one of sample local medical images corresponding to the plurality of axial slices or the plurality of tissue regions are obtained. The sample medical images can be global images. Based on identification information of the sample medical images, the computer device can directly obtain at least one of the sample local medical images corresponding to different axial slices and the sample local medical images corresponding to different tissue regions corresponding to the identification information from a PACS system. Alternatively, the computer device can also segment the sample medical images in an axial dimension to obtain sample local medical images corresponding to different axial slices. Furthermore, the computer device can segment the sample medical images in a horizontal dimension to obtain sample local medical images corresponding to different tissue regions.

In a step S402, a noise level of the sample local medical images is predicted using a noise prediction model to obtain noise level information. The noise prediction model includes at least one of an axial slice noise prediction model and a regional noise prediction model.

In some embodiments, a computer device may use sample local medical images corresponding to different axial slices as input signals and input them into an axial slice noise prediction model. The axial slice noise prediction model may extract feature information from the sample local medical images corresponding to the axial slices and perform a noise prediction based on extracted feature information to obtain axial slice noise level information. The axial slice noise level information may include noise level information corresponding to the sample local medical images corresponding to the axial slices.

In some embodiments, a computer device may use sample local medical images corresponding to different tissue regions as input signals and input them into a regional noise prediction model. The regional noise prediction model may extract feature information from the sample local medical images corresponding to the tissue regions and perform a noise prediction based on extracted feature information to obtain regional noise level information. The regional noise level information may include noise level information corresponding to the sample local medical images corresponding to the tissue regions.

In some embodiments, the noise level information may include at least one of axial slice noise level information and regional noise level information.

In a step S403, the noise level information are fused with the sample medical images to obtain fused sample medical images.

Specifically, the quantified noise level values and corresponding pixel values of the sample medical images can be superimposed based on predetermined weights to obtain the fused sample medical images.

In a step S404, an initial denoising model is trained using the fused sample medical images until an output result of the initial denoising model meets a predetermined condition, to obtain the denoising model.

In some embodiments, after obtaining the noise level information, the computer device can input at least one of the axial slice noise level information and the regional noise level information, combined with the fused sample medical images, into the initial denoising model. The initial denoising model can perform denoising on the sample local medical images corresponding to different axial slices and the sample local medical images corresponding to different tissue regions, and output the target medical image obtained by a current denoising. It is determined whether the target medical image obtained by the current denoising meets the predetermined condition. If not, the parameter information of the initial denoising model is adjusted until an output result of the initial denoising model meets a predetermined condition. At this time, it indicates that the training process of the initial denoising model is completed, a denoising effect of the initial denoising model is good, and a trained initial denoising model is used as the denoising model.

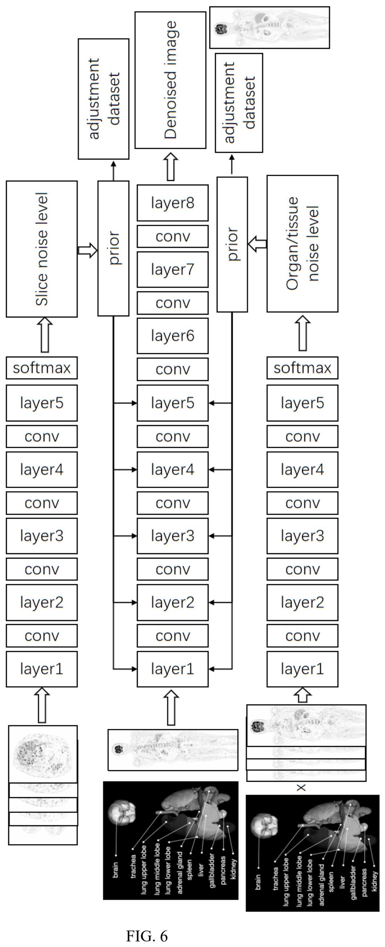

As shown in FIG. 6, this is a schematic flowchart of an overall process of adaptive denoising using a noise-aware network to assist a denoising network. CT images and PET images can be registered and fused, which enables obtaining specific positions of tissues or organs in the PET images. The fused PET-CT images are input into the denoising network for training. The noise-aware network includes an axial slice noise-aware branch and an organ/tissue noise-aware branch. The axial slice noise-aware branch performs feature extraction and noise evaluation on local medical images corresponding to different axial slices through a convolutional neural network to output a slice noise level. The organ/tissue noise-aware branch processes a fused input of anatomically annotated medical images and initial medical images through a convolutional neural network to output an organ/tissue noise level. The aforementioned information of noise levels, serving as prior information, is fused with the inputs of some layers in the denoising network. The noise information can be either the noise level images at different positions finally output by the noise-aware network or its intermediate slice feature information, and can be optimized for fusion through value range scaling or smoothing. Fusion methods include adding and concatenating with the feature inputs of some layers of the denoising network, connecting via some neural network layers, or designing a transformation matrix to transform the original feature inputs of some layers based on the noise information. Ultimately, a denoised image is output and an adjustment dataset is generated.

The most commonly used brain slices are three standard orthogonal directions: axial , coronal, and sagittal, which are mutually perpendicular and can cover most basic diagnostic needs without oblique cutting. When it is necessary to focus on observing special structures of the brain in non-orthogonal directions, an oblique cutting scheme will be customized, to make the target structure completely present in the same section, avoiding being cut off by orthogonal sections.

The training process of the above-mentioned denoising model specifically obtains sample local medical images corresponding to axial slices or tissue regions, and matches the corresponding type of noise prediction model for noise prediction. Compared with the traditional global unified noise processing method, it can more accurately capture the noise characteristics of different local areas and effectively avoid noise prediction deviation. Then, the accurate noise level information are integrated with the sample medical images to provide richer noise information support for the initial denoising model. Combined with local medical images for joint training, the model can take into account both global and local denoising needs, significantly improving the detail retention (such as lesion edges, tissue texture) and noise removal effect of the medical images after denoising. The final denoising model can adapt to medical image denoising scenarios of different axial slices and different tissue regions.

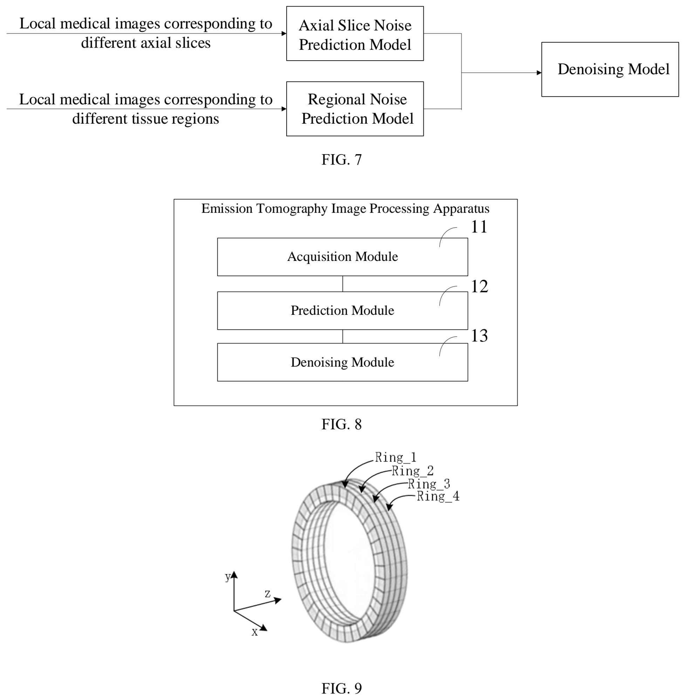

FIG. 7 is a schematic diagram of models of a noise reduction process, which includes three models: an axial slice noise prediction model, a regional noise prediction model, and a denoising model. The axial slice noise prediction model takes as input local medical images corresponding to different axial slices, and outputs quantified values of the noise level for each axial slice. The regional noise prediction model takes as input local medical images corresponding to different tissue regions, and outputs quantified values of the noise level for each tissue region. The denoising model takes as input the outputs of the axial slice noise prediction model and the regional noise prediction model, and outputs the target medical image after denoising.

During the denoising model training process, the noise level prediction results of the axial slice noise prediction model are processed and used as prior information, and the noise level prediction results of the regional noise prediction model are processed and used as prior information. These two types of prior information are spliced and input into the denoising model for training, resulting in a trained denoising model. This denoising model can suppress axial noise and noise in different tissue regions. These two types of prior information can also be used to adjust the sample dataset, changing the mapping relationship between the input image and output image of the denoising model, thereby improving the denoising effect of the target medical image after denoising.

It should be understood that, although the steps in the flowcharts of the above embodiments are shown in sequence as indicated by the arrows, these steps are not necessarily performed in the order indicated by the arrows.

Unless otherwise specified herein, there is no strict order restriction on the execution of these steps, and these steps can be performed in other orders. Moreover, at least a portion of the steps in the flowcharts of the above embodiments may include a plurality of steps or a plurality of stages, and these steps or stages are not necessarily performed at the same time, but can be performed at different times. The execution order of these steps or stages is not necessarily to be performed in sequence, but can be performed in turn or alternately with other steps or at least a portion of steps or stages in other steps.

Based on the same inventive concept, an emission tomography image processing apparatus, which is for implementing the aforementioned emission tomography image processing method, is further provided in the embodiments of the present disclosure. The solution provided by this apparatus is similar to the solution described in the aforementioned method. Therefore, the specific limitations of one or more embodiments of the emission tomography image processing apparatus provided below can be found in the aforementioned limitations of the emission tomography image processing method and are not further elaborated here.

In an exemplary embodiment, as shown in FIG. 8, an emission tomography image processing apparatus is provided, including an acquisition module 11, a prediction module 12, and a denoising module 13.

The acquisition module 11 is configured to acquire a local medical image of a target object, where different local medical images correspond to different spatial positions or different tissue regions.

The prediction module 12 is configured to predict noise level information corresponding to the local medical images.

The denoising module 13 is configured to perform image denoising on the local medical image based on the noise level information corresponding to the local medical images to obtain a target medical image, where at least two of the local medical images correspond to different noise level information.

In an exemplary embodiment, the acquisition module 11 includes an axial dimension division unit.

The axial dimension division unit is configured to acquire local medical images of the target object corresponding to different axial slices.

In an exemplary embodiment, the prediction module 12 includes an axial slices prediction unit.

The axial slice prediction unit is configured to input the local medical images corresponding to the axial slices into an axial slice noise prediction model, and predict a noise level of the local medical images corresponding to the different axial slices using the axial slice noise prediction model to obtain noise level information corresponding to the different axial slices.

In an exemplary embodiment, the acquisition module 11 further includes a horizontal dimension division unit.

The horizontal dimension division unit is configured to acquire local medical images of the target object corresponding to different tissue regions.

In an exemplary embodiment, the prediction module 12 includes a tissue region prediction unit.

The tissue region prediction unit is configured to input local medical images corresponding to the tissue regions into a regional noise prediction model, and predict a noise level of the local medical images corresponding to different tissue regions using the regional noise prediction model to obtain noise level information corresponding to the different tissue regions.

In an exemplary embodiment, the denoising module 13 includes a denoising unit.

The denoising unit is configured to perform image denoising on the local medical image based on the noise level information corresponding to the local medical images using a predetermined denoising model to obtain the target medical image.

In an exemplary embodiment, the image denoising apparatus further includes: a sample image acquisition module, a noise prediction module, a fusion module, and a training module.

The sample image acquisition module is configured to acquire sample medical images and sample local medical images of a sample object. The sample local medical images include at least one of sample local medical images corresponding to a plurality of axial slices and sample local medical images corresponding to a plurality of tissue regions.

The noise prediction module is configured to predict a noise level of the sample local medical images using a noise prediction model to obtain noise level information. The noise prediction model includes at least one of an axial slice noise prediction model and a regional noise prediction model.

The fusion module is configured to fuse the noise level information with the sample medical images to obtain fused sample medical images.

The training module is configured to train the initial denoising model using the fused sample medical images until an output result of the initial denoising model meets a predetermined condition, to obtain the denoising model.

The emission tomography image processing apparatus described above may be implemented in whole or in part through software, hardware, or a combination thereof. Each module may be embedded in or independent of a processor in a computer device in the form of hardware, or may be stored in a computer device memory in the form of software, so that the processor can call and execute the corresponding operations of each module.

In an exemplary embodiment, a computer device is provided, including a memory and a processor. The memory stores a computer program, and the processor implements the steps of the above-mentioned emission tomography image processing method in any embodiment when executing the computer program.

In some embodiments, a non-transitory computer-readable storage medium is provided, on which a computer program is stored. When the computer program is executed by a processor, the steps of the above-mentioned emission tomography image processing method in any embodiment is implemented.

In some embodiments, a computer program product is provided, including a computer program, which implements the steps of the above-mentioned emission tomography image processing method in any one embodiment when executed by a processor.

It should be noted that the user information (including but not limited to user device information, user personal information, etc.) and data (including but not limited to data used for analysis, stored data, displayed data, etc.) involved in the present disclosure are all information and data authorized by the user or fully authorized by all parties, and the collection, use and processing of relevant data must comply with relevant regulations.

It should be noted that in the 3D acquisition mode of PET (Positron Emission Tomography), a sensitivity of an axial edge region is lower than that of an axial center region. Therefore, a counting level of the axial edge region is lower than that of the axial center region.

In a statistical-data-based iterative reconstruction algorithm, the low count level in the axial edge region will cause overfitting of Poisson noise, amplifying the image noise level in the axial edge region, resulting in the image noise level in the axial edge region being higher than the image noise level in the axial center region.

The detector used in the PET system includes a plurality of detection rings arranged in parallel. In the example shown in FIG. 9, the detector includes four detection rings, denoted as Ring_1, Ring_2, Ring_3, and Ring_4, and each detection ring is composed of a number of detection units.

A direction in which the detector rings are arranged side by side can be called the axial direction, and a plane perpendicular to the axial direction can be called the cross-section. An axial direction can be denoted as z, and two mutually perpendicular directions can be defined on the cross-section, denoted as x and y.

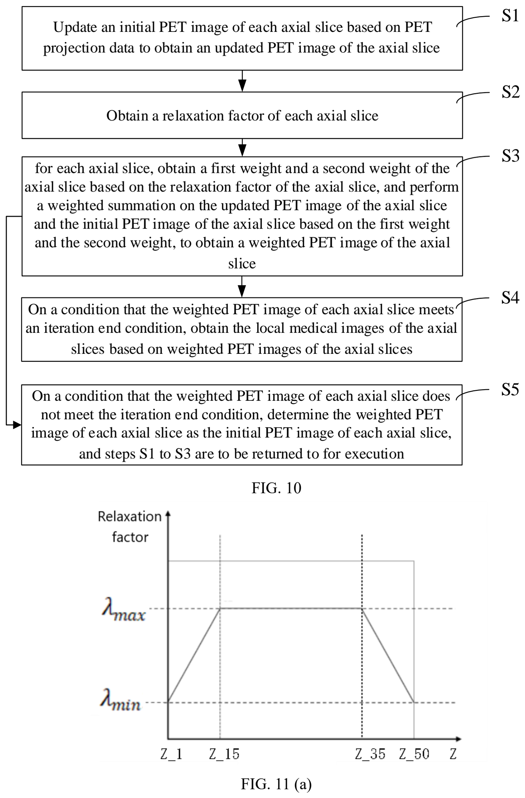

A PET image reconstruction method that can be executed by a computer device is provided in the present disclosure, including the steps shown in FIG. 10. The PET image reconstruction method can be used to obtain local medical images corresponding to different axial slices of a target object, further reducing the noise level of the image.

In a step S1, an initial PET image of each axial slice is updated based on PET projection data to obtain an updated PET image of the axial slice.

In PET imaging, any two detector elements in a detector form a line of response (LOR). A number of detector elements in a detector can be determined by a number of detection rings and a number of detection elements in each detection ring.

After performing a PET scan on a target object, a number of events detected by each line of response (LOR) is obtained. These events form the PET projection data, which are projection data acquired during the scan. For example, on a condition that there are m LORs, the PET projection data include $\{y_{LOR\_1}, y_{LOR\_2}, ..., y_{LOR\_m}\}$, where $y_{LOR\_m}$ refers to the number of events detected by m-th LOR. The PET projection data can be expressed in a matrix format with m rows and one column, denoted by Y.

Different axial slices correspond to different axial positions. For example, two adjacent axial slices correspond to axial positions z_i and z_i+1, respectively. These two axial slices can be denoted as $slice_{z\_i}$ and $slice_{z\_i+1}$. Among the axial slices, the axial slice that is aligned or nearly aligned with the axial position of a central detection ring is a central axial slice. The central detection ring can be a detection ring in the middle of a plurality of parallel detection rings.

Taking the axial $slice_{z\_i}$ as an example, the explanation is as follows.

On a condition that a current iteration is a first iteration, the initial PET image (which can be denoted as $I^1_{z\_i}$) of the axial $slice_{z\_i}$ in the first iteration can be randomly generated. The initial PET image $I^1_{z\_i}$ is updated based on the PET projection data, an updated PET image (which can be denoted as $I'^1_{z\_i}$) of the axial $slice_{z\_i}$ in the first iteration is obtained.

On a condition that the current iteration is not the first iteration, the current iteration is denoted as a k-th iteration, where k refers to a positive integer greater than or equal to 2. Then, a weighted PET image of the axial $slice_{z\_i}$ obtained in k-1-th iteration can be obtained, and the PET image is used as the initial PET image (which can be denoted as $I^k_{z\_i}$) of the axial $slice_{z\_i}$ in the k-th iteration. $\bar{I}^k_{z\_i}$ is updated based on the PET projection data, an updated PET image (which can be denoted as $I'^k_{z\_i}$) of the axial $slice_{z\_i}$ in the k-th iteration is obtained.

According to the processing method of the axial $slice_{z\_i}$, other axial slices can be processed to obtain updated PET images of other axial slices in each iteration.

In a step S2, a relaxation factor of each axial slice is obtained.

In the present disclosure, corresponding relaxation factors for different axial slices are set, which can control a convergence degree of different axial slices and avoid image edge noise enhancement due to excessive convergence.

In a step S3, for each axial slice, a first weight and a second weight of the axial slice are obtained based on the relaxation factor of the axial slice, and a weighted summation is performed on the updated PET image of the axial slice and the initial PET image of the axial slice based on the first weight and the second weight, to obtain a weighted PET image of the axial slice.

Taking the axial slice$_{z\_i}$ as an example, the explanation is as follows.

On a condition that the current iteration is a first iteration, an updated PET image $I'^{1}_{z\_i}$ of the axial slice$_{z\_i}$ in the first iteration can be obtained through step S1. Based on the relaxation factor of the axial slice$_{z\_i}$, a first weight and a second weight of the axial slice$_{z\_i}$ can be obtained. Based on the first weight and the second weight of the axial slice$_{z\_i}$, a weighted summation is performed on the updated PET image $I'^{1}_{z\_i}$ of the axial slice$_{z\_i}$ in the first iteration and the initial PET image $I^{1}_{z\_i}$ of the axial slice$_{z\_i}$ in the first iteration to obtain a weighted PET image of the axial slice$_{z\_i}$ obtained in the first iteration. On a condition that the axial slice$_{z\_i}$ is an axial slice within a predetermined range, the closer the axial slice$_{z\_i}$ is to the central axial slice, the greater the relaxation factor of the axial slice$_{z\_i}$, the greater the weight assigned to the updated PET image $I'^{1}_{z\_i}$ of the axial slice$_{z\_i}$ in the first iteration, and the smaller the weight assigned to the initial PET image $I^{1}_{z\_i}$ of the axial slice in the first iteration.

On a condition that the current iteration is not the first iteration, the current iteration is denoted as the k-th iteration. Then, through step S1, the updated PET image $I'^{k}_{z\_i}$ of the axial slice$_{z\_i}$ in the k-th iteration is obtained. Based on the relaxation factor of the axial slice$_{z\_i}$, the first weight and the second weight of the axial slice$_{z\_i}$ can be obtained. Based on the first weight and the second weight of the axial slice$_{z\_i}$, a weighted summation is performed on the updated PET image $I'^{k}_{z\_i}$ of the axial slice$_{z\_i}$ in the k-th iteration and the initial PET image $I^{k}_{z\_i}$ of the axial slice$_{z\_i}$ in the k-th iteration to obtain the weighted PET image of the axial slice$_{z\_i}$ in the k-th iteration. On a condition the axial slice$_{z\_i}$ is an axial slice within a predetermined range, the closer the axial slice$_{z\_i}$ is to the central axial slice, the greater the relaxation factor of the axial slice$_{z\_i}$, the greater the weight assigned to the updated PET image $I'^{k}_{z\_i}$ of the axial slice$_{z\_i}$ in the k-th iteration, and the smaller the weight assigned to the initial PET image $I^{k}_{z\_i}$ of the axial slice$_{z\_i}$ in the k-th iteration.

According to the processing method of the axial slice$_{z\_i}$, other axial slices can be processed to obtain weighted PET image of other axial slices obtained in each iteration.

In a step S4, on a condition that the weighted PET image of each axial slice meets an iteration end condition, the local medical images corresponding to the axial slices are obtained based on weighted PET images of the axial slices.

Taking the axial slice$_{z\_i}$ as an example, the explanation is as follows.

On a condition that the current iteration is the first iteration, the weighted PET image of the axial slice$_{z\_i}$ obtained in the first iteration can be obtained through step S2. On a condition that the weighted PET image of each axial slice obtained in the first iteration meets the iteration end condition, the weighted PET image of the axial slice$_{z\_i}$ obtained in the first iteration is determined as the local medical image of the axial slice$_{z\_i}$.

On a condition that the current iteration is not the first iteration, the current iteration is denoted as the k-th iteration, and the weighted PET image of the axial slice$_{z\_i}$ obtained in the k-th iteration can be obtained through step S2. On a condition that the weighted PET image of each axial slice obtained in the k-th iteration meets the iteration end condition, the weighted PET image of the axial slice$_{z\_i}$ obtained in the k-th iteration is determined as the local medical image of the axial slice$_{z\_i}$.

According to the processing method of the axial slice$_{z\_i}$, other axial slices can be processed to obtain local medical images of other axial slices.

In a step S5, on a condition that the weighted PET image of each axial slice does not meet the iteration end condition, the weighted PET image of each axial slice is determined as the initial PET image of each axial slice, and steps S1 to S3 are to be returned to for execution.

Taking the axial slice$_{z\_i}$ as an example, the explanation is as follows.

On a condition that the current iteration is the first iteration, the weighted PET image of the axial slice$_{z\_i}$ obtained in the first iteration can be obtained through step S2. if the weighted PET image of each axial slice obtained in the first iteration do not meet the iteration end condition, the weighted PET image of the axial slice$_{z\_i}$ obtained in the first iteration is determined as the initial PET image of the axial slice$_{z\_i}$ in the second iteration.

On a condition that the current iteration is not the first iteration, the current iteration is denoted as the k-th iteration, and the weighted PET image of the axial slice$_{z\_i}$ obtained in the k-th iteration can be obtained through step S2. On a condition that the weighted PET image of each axial slice obtained in the k-th iteration meets the iteration end condition, the weighted PET image of the axial slice$_{z\_i}$ obtained in the k-th iteration is determined as the initial PET image of the axial slice$_{z\_i}$ in the k+1-th iteration, and steps S1 to S3 are to be returned to for execution.

According to the processing method of the axial slice$_{z\_i}$, other axial slices can be processed to obtain initial PET images of other axial slices in the next iteration, and steps S1 to S3 are to be returned to for execution.

In the above-mentioned PET image reconstruction method, in the step S1, an initial PET image of each axial slice is updated based on PET projection data to obtain an updated PET image of the axial slice. In the step S2, a relaxation factor of each axial slice is obtained. In the step S3, for each axial slice, a first weight and a second weight of the axial slice are obtained based on the relaxation factor of the axial slice, and a weighted summation is performed on the updated PET image of the axial slice and the initial PET image of the axial slice based on the first weight and the second weight, to obtain a weighted PET image of the axial slice. In the step S4, on a condition that the weighted PET image of each axial slice meets an iteration end condition, the local medical images corresponding to the axial slices are obtained based on weighted PET images of the axial slices. In the step S5, on a condition that the weighted PET image of each axial slice does not meet the iteration end condition, the weighted PET image of each axial slice is determined as the initial PET image of each axial slice, and the steps S1 to S3 are to be returned to for execution. With the help of the solution provided by the present disclosure, the image noise level in the axial edge region can be suppressed during the iterative reconstruction process to avoid excessive convergence leading to an increase in the image noise level in the axial edge region.

In some embodiments, obtaining the relaxation factors of the axial slices includes:

For each axial slice, a slice number difference between the axial slice and a central axial slice is obtained, and on a condition that the slice number difference is greater than a slice number difference threshold, the relaxation factor of the axial slice is determined based on the slice number difference and a predetermined mapping relationship. The relaxation factor of the axial slice is smaller than a relaxation factor of the central axial slice.

Taking axial slice$_{z\_i}$ as an example, slice$_{z\_i}$ can be subtracted from the central axial slice, and a subtraction result is used as the slice number difference. On a condition that the slice number difference is greater than a slice number difference threshold, the relaxation factor of axial slice$_{z\_i}$ can be determined based on the slice number difference and a predetermined mapping relationship, and the relaxation factor of axial slice$_{z\_i}$ is smaller than the relaxation factor of the central axial slice. The predetermined mapping relationship may include a negative correlation function, which describes the negative correlation between the slice number difference and the relaxation factor. Therefore, the larger the slice number difference, the smaller the relaxation factor determined by the negative correlation function.

In some embodiments, the method provided herein further includes:
on a condition that the slice number difference is less than or equal to the slice number difference threshold, determining the relaxation factor of the central axial slice as the relaxation factor of the axial slice.

Taking the axial slice$_{z\_i}$ as an example, slice$_{z\_i}$ can be subtracted from the central axial slice, and the subtraction result can be used as the slice number difference. On a condition that the slice number difference is less than or equal to the slice number difference threshold, the relaxation factor of the central axial slice is determined as the relaxation factor of the axial slice$_{z\_i}$.

Specifically, a curve of relaxation factor versus axial slice may be set, where the curve describes values of relaxation factors of different axial slices.

In the example shown in FIG. 11(a), there are 50 axial slices. The central axial slice corresponds to the axial position z_25 and can be denoted as slice$_{z\_25}$. The axial slice corresponding to the axial position z_15 is denoted as slice$_{z\_15}$, and the axial slice corresponding to the axial position z_35 is denoted as slice$_{z\_35}$. The axial slices between slice$_{z\_15}$ and slice$_{z\_30}$ are axial slices close to the central axial slice. In the example shown in FIG. 11(a), the slice number difference threshold is set to 10.

In the axial slices between slice$_{z\_15}$ and slice$_{z\_50}$, the relaxation factor of each non-central axial slice is the relaxation factor of the central axial slice$_{z\_25}$. The relaxation factor of the axial slice$_{z\_25}$ is a predetermined maximum value, denoted as $\lambda_{max}$.

In the axial slices between slice$_{z\_1}$ and slice$_{z\_15}$, the relaxation factor of each axial slice is taken according to the following negative correlation function: $\lambda_{z\_j} = k_1 j + \lambda_{min}$, where j refers to a positive integer between 1 and 15, $k_1$ belongs to a predetermined value, $k_1$ refers to a value greater than 0, $\lambda_{min}$ belongs to a predetermined value, $\lambda_{min}$ refers to a value greater than 0, and $\lambda_{z\_j}$ refers to the relaxation factor of the axial slice$_{z\_j}$.

In the axial slices between slice$_{z\_35}$ and slice$_{z\_50}$, the relaxation factor of each axial slice is taken according to the following negative correlation function: $\lambda_{z\_q} = k_2 q + b$, where q refers to a positive integer between 35 and 50, $k_2$ belongs to a predetermined value, $k_2$ refers to a value less than 0, b belongs to a predetermined value, b refers to a value greater than 0, and $\lambda_{z\_q}$ refers to the relaxation factor of the axial slice$_{z\_q}$.

In this embodiment, for any axial slice within a predetermined range (e.g., an axial slice between slice$_{z\_1}$ and slice$_{z\_15}$ or an axial slice between slice$_{z\_35}$ and slice$_{z\_50}$), the closer the axial slice is to the central axial slice, the greater the relaxation factor of the axial slice. Accordingly, the weight assigned to the weighted PET image of the axial slice obtained in each iteration is greater, and the weight assigned to the initial PET image of the axial slice in each iteration is smaller. This allows suppressing the image noise level in the axial edge region during the iterative reconstruction process.

In some embodiments, obtaining the relaxation factors of the axial slices includes:

obtaining, for each axial slice, a slice number difference between the axial slice and a central axial slice; and determining the relaxation factor of the axial slice based on the slice number difference and a predetermined mapping relationship.

Taking axial slice$_{z\_i}$ as an example, slice$_{z\_i}$ can be subtracted from the central axial slice, and the subtraction result can be used as the slice number difference. Based on the slice number difference and a predetermined mapping relationship, the relaxation factor of axial slice$_{z\_i}$ is determined, and the relaxation factor of axial slice$_{z\_i}$ is smaller than the relaxation factor of the central axial slice. The predetermined mapping relationship may include a negative correlation function, which describes the negative correlation between the slice number difference and the relaxation factor. Therefore, the larger the slice number difference, the smaller the relaxation factor determined by the negative correlation function.

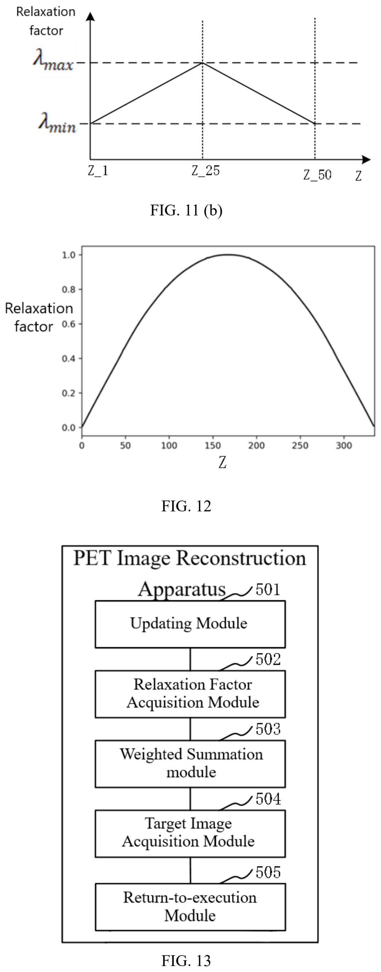

In the example shown in FIG. 11(b), there are 50 axial slices. The central axial slice is the axial slice corresponding to the axial position z_25, which can be denoted as slice$_{z\_25}$. The relaxation factor of the axial slice$_{z\_25}$ is the predetermined maximum value, denoted as $\lambda_{max}$.

In the axial slices between slice$_{z\_1}$ and slice$_{z\_24}$, the relaxation factor of each axial slice is taken according to the following negative correlation function: $\lambda_{z\_p} = k_3 p + \lambda_{min}$, where p refers to a positive integer between 1 and 24, $k_3$ refers to a predetermined value, $k_3$ refers to a value greater than 0, $\lambda_{min}$ refers to a predetermined value, $\lambda_{min}$ refers to a value greater than 0, and $\lambda_{z\_p}$ refers to the relaxation factor of the axial slice$_{z\_p}$.

In the axial slices between slice$_{z\_26}$ and slice$_{z\_50}$, the relaxation factor of each axial slice is taken according to the following negative correlation function: $\lambda_{z\_h} = k_4 h + a$, where h refers to a positive integer between 26 and 50, $k_4$ belongs to a predetermined value, $k_4$ refers to a value less than 0, a belongs to a predetermined value, a refers to a value greater than 0, and $\lambda_{z\_h}$ refers to the relaxation factor of the axial slice$_{z\_h}$.

In this embodiment, for any axial slice within a predetermined range (e.g., axial slices between slice$_{z\_1}$ and slice$_{z\_50}$), the closer the axial slice is to the central axial slice, the greater the relaxation factor of the axial slice. Accordingly, the weight assigned to the weighted PET image of the axial slice obtained in each iteration is greater, and the weight assigned to the initial PET image of the axial slice in each iteration is smaller. This allows suppressing the image noise level in the axial edge region during the iterative reconstruction process.

In some embodiments, obtaining the relaxation factors of the axial slices includes:
for each voxel on the axial slice, obtaining a detection probability of each line of response (LOR) for the voxel, obtaining an attenuation coefficient of each LOR, and obtaining, for each axial slice, the relaxation factor of the axial slice based on the detection probability of each LOR of each voxel on the axial slice and the attenuation coefficient of each LOR of each voxel.

Exemplarily, a number of voxels is n and a number of LORs is m, and n voxels can be respectively denoted as: v_1, v_2, v_3, . . ., and v_n.

When the number of LORs is m and the number of voxels is n, an orthographic projection model of a PET system can be expressed as: Y = PI. The orthographic projection model is expanded as:

$$\begin{bmatrix} y_{LOR\_1} \\ y_{LOR\_2} \\ \cdots \\ y_{LOR\_m} \end{bmatrix} = \begin{bmatrix} p_{(LOR\_1,v\_1)} & p_{(LOR\_1,v\_2)} & \cdots & p_{(LOR\_1,v\_n)} \\ p_{(LOR\_2,v\_1)} & p_{(LOR\_2,v\_2)} & \cdots & p_{(LOR\_2,v\_n)} \\ \cdots & \cdots & \cdots & \cdots \\ p_{(LOR\_m,v\_1)} & p_{(LOR\_m,v\_2)} & \cdots & p_{(LOR\_m,v\_n)} \end{bmatrix} \times \begin{bmatrix} i_{v\_1} \\ i_{v\_2} \\ \cdots \\ i_{v\_n} \end{bmatrix},$$

where Y refers to acquired scanned PET projection data, P refers to a system response matrix, and I refers to a PET image.

Y includes the number of events detected by m LORs, denoted as $y_{LOR\_1}$, $y_{LOR\_2}$, $y_{LOR\_3}$, ..., and $y_{LOR\_m}$. P includes the probability of detection for each voxel by m LORs, $p_{(LOR\_m.v\_n)}$ refers to the probability that the m-th LOR will detect the n-th voxel. I includes tracer concentration values for each of n voxels, denoted as iv_1, iv_2, iv_3, ..., and iv_n.

For each voxel, such as voxel v_1, the detection probability of each LOR for voxel v_1 can be obtained in the system response matrix, i.e., $\{p_{(LOR\_1,v\_1)}, p_{(LOR\_2,v\_1)},..., p_{(LOR\_m,v\_1)}\}$. In addition, the attenuation coefficient of each of m LORs can also be obtained, denoted as $\{k_{LOR\_1}, k_{LOR\_2},..., k_{LOR\_m}\}$. An attenuation coefficient of each LOR can be obtained from a forward projection of an attenuation image.

On a condition that among n voxels, the voxels located in the axial slice$_{z\_i}$ include v_1 to v_5, the relaxation factor of the axial slice$_{z\_i}$ can be obtained based on the detection probability $\{p_{(LOR\_1,v\_1)}, p_{(LOR\_2,v\_1)},..., p_{(LOR\_m,v\_1)}\}$ of each LOR for voxel v_1, the detection probability $\{p_{(LOR\_1,v\_2)}, p_{(LOR\_2,v\_2)},..., p_{(LOR\_m,v\_2)}\}$ of each LOR for voxel v_2, the detection probability $\{p_{(LOR\_1,v\_3)}, p_{(LOR\_2,v\_3)},..., p_{(LOR\_m,v\_3)}\}$ of each LOR for voxel v_3, the detection probability $\{p_{(LOR\_1,v\_4)}, p_{(LOR\_2,v\_4)},..., p_{(LOR\_m,v\_4)}\}$ of each LOR for voxel v_4, the detection probability $\{p_{(LOR\_1,v\_5)}, p_{(LOR\_2,v\_5)},..., p_{(LOR\_m,v\_5)}\}$ of each LOR for voxel v_5, and the attenuation coefficients $\{k_{LOR\_1}, k_{LOR\_2},..., k_{LOR\_m}\}$ of each of the m LORs.

In this embodiment, the relaxation factor of each axial slice is obtained by combining the detection probability of each LOR for a voxel and the attenuation coefficient of each LOR. During the iterative reconstruction process, the image noise level in the axial edge region is better suppressed.

In some embodiments, for each axial slice, obtaining the relaxation factor of the axial slice based on the detection probability of each LOR of each voxel on the axial slice and the attenuation coefficient of each LOR of each voxel, includes: for each voxel on the axial slice, performing a weighted summation on the detection probability of each LOR of the voxel and the attenuation coefficient of each LOR to obtain a detection probability weighted value of the voxel, and obtaining the relaxation factor of the axial slice based on the detection probability weighted value of each voxel on the axial slice.

Continuing with the previous example, in which voxels located in axial slice$_{z\_i}$ include v_1 through v_5, a weighted summation is performed on the detection probability $\{p_{(LOR\_1,v\_1)}, p_{(LOR\_2,v\_1)}, ..., p_{(LOR\_m,v\_1)}\}$ for each LOR for voxel v_1 and the attenuation coefficients $\{k_{LOR\_1}, k_{LOR\_2}, ..., k_{LOR\_m}\}$ for each of the m LORs. Specifically, the attenuation coefficient of a particular LOR is used as a weight to assign the detection probability of that LOR for voxel v_1 to obtain a weighted value for the detection probability of each LOR for voxel v_1. Based on the weights, a weighted summation is performed on the detection probability $\{p_{(LOR\_1,v\_1)}, p_{(LOR\_2,v\_1)}, ..., p_{(LOR\_m,v\_1)}\}$ for each LOR for voxel v_1, which can be expressed as: $p_{(LOR\_1,v\_1)} \times k_{LOR\_1} + p_{(LOR\_2,v\_1)} \times k_{LOR\_2} + ... + p_{(LOR\_m,v\_1)} \times k_{LOR\_m}$. A weighted summation result can be used as the detection probability weighted value of voxel v_1.

In the above manner, the detection probability weighted value of voxel v_2, the detection probability weighted value of voxel v_3, the detection probability weighted value of voxel v_4, and the detection probability weighted value of voxel v_5 can also be obtained.

A relaxation factor of the axial slice$_{z\_i}$ can be obtained by adding the detection probability weighted value of voxel v_1, the detection probability weighted value of voxel v_2, the detection probability weighted value of voxel v_3, the detection probability weighted value of voxel v_4 and the detection probability weighted value of voxel v_5.

Referring to FIG. 12, a curve shown in FIG. 12 refers to the relaxation factor of each axial slice obtained in the above-described manner. For any axial slice shown in FIG. 12, the closer the axial slice is to the central axial slice, the larger the relaxation factor of the axial slice. Accordingly, the weight assigned to the weighted PET image of the axial slice obtained in each iteration of is larger, and the weight assigned to the initial PET image of the axial slice in each iteration is smaller, thereby suppressing the image noise level in the axial edge region during the iterative reconstruction process.

In this embodiment, for a certain voxel, the attenuation coefficient of the LOR is used as the weight of the detection probability of the LOR for the voxel, and a weighted summation is performed on the detection probability of each LOR for the voxel to obtain the detection probability weighted value of the voxel, so as to obtain the relaxation factor of the corresponding axial slice. During the iterative reconstruction process, the image noise level in the axial edge region is better suppressed.

In some embodiments, obtaining the first weight and the second weight of the axial slice based on the relaxation factor of the axial slice includes:

obtaining the first weight of the axial slice based on the relaxation factor of the axial slice, and obtaining the second weight of the axial slice based on a difference between a predetermined value and the relaxation factor of the axial slice.

The axial slice has two weights. A sum of these two weights must meet certain requirements. A value that meets the requirements can be used as the predetermined value. In some scenarios, the sum of these two weights must be equal to 1, in which case 1 can be used as the predetermined value.

Taking axial slice$_{z\_i}$ as an example, the relaxation factor of axial slice$_{z\_i}$ can be obtained and used as the first weight of axial slice$_{z\_i}$. The predetermined value is subtracted from the relaxation factor of axial slice$_{z\_i}$, and the difference is used as the second weight of axial slice$_{z\_i}$. Using this processing method, the first weight of axial slice$_{z\_i}$ can be subsequently assigned to the weighted PET image of the axial slice$_{z\_i}$ obtained in each iteration. The second weight of axial slice$_{z\_i}$ can be assigned to the initial PET image of axial slice$_{z\_i}$ in each iteration.

In some embodiments, updating the initial PET image of each axial slice based on the PET projection data to obtain the updated PET image of each axial slice includes:

obtaining first PET projection prediction data based on an initial PET image of each axial slice, and updating the initial PET image of each axial slice based on differences between the first PET projection prediction data and the PET projection data to obtain the updated PET image of each axial slice.

Based on the initial PET image of each axial slice, PET projection prediction data can be obtained. This PET projection prediction data are obtained by prediction and, for purposes of distinction, are referred to as the first PET projection prediction data. On a condition that the initial PET image of each axial slice is close to a true image, the first PET projection prediction data will be closer to acquired scanned PET projection data. Therefore, based on the difference between the first PET projection prediction data and the PET projection data, the initial PET image of each axial slice can be updated to obtain the updated PET image of each axial slice.

For example, on a condition that the current iteration is the first iteration, an initial PET image for each axial slice in the first iteration can be randomly generated. Based on the initial PET image for each axial slice in the first iteration and the acquired scanned PET projection data, image update information for the first iteration can be obtained, which can be denoted as $I_{update}^1$. The image update information $I_{update}^1$ for the first iteration reflects the difference between the first PET projection prediction data and the PET projection data.

The specific formula for obtaining the image update information for the first iteration is as follows:

$$I_{update}^1 = \frac{P^T \cdot \frac{Y}{PI^1}}{P^T 1}$$, where P refers to the system response matrix, Y refers to the acquired scanned PET projection data, $I^1$ includes the initial PET image of each axial slice in the first iteration, $P^T$ refers to a transposed matrix of the system response matrix, and 1 refers to an all-ones matrix.

Taking the axial slice$_{z\_i}$ as an example, after obtaining the image update information $I_{update}^1$ for the first iteration, the initial PET image $I_{z\_i}^1$ of the axial slice$_{z\_i}$ in the first iteration can be updated based on the image update information $I_{update}^1$ for the first iteration to obtain the updated PET image $I'^1_{z\_i}$ of the axial slice$_{z\_i}$ in the first iteration.

Specifically, the image update information $I_{update}^1$ for the first iteration and the initial PET image $I_{z\_i}^1$ of the axial slice$_{z\_i}$ in the first iteration are multiplied, and a product result is used as the updated PET image $I'^1_{z\_i}$ of the axial slice$_{z\_i}$ in the first iteration.

Similarly, the initial PET images of other axial slices in the first iteration may be updated based on the image update information $I_{update}^1$ for the first iteration to obtain updated PET images of other axial slices in the first iteration.

In another example, on a condition that the current iteration is not the first iteration, the current iteration is denoted as the k-th iteration. For each axial slice, a weighted PET image of the axial slice obtained in the k-1-th iteration can be obtained, and this PET image can be used as the initial PET image of the axial slice in the k-th iteration. Based on the initial PET image of each axial slice in the k-th iteration and the acquired scanned PET projection data, image update information for the k-th iteration can be obtained and denoted as $I_{update}^k$. The image update information $I_{update}^k$ for the k-th iteration reflects the difference between the first PET projection prediction data and the PET projection data.

The specific formula for obtaining the image update information for the k-th iteration is as follows:

$$I_{update}^k = \frac{P^T \cdot \frac{Y}{PI^k}}{P^T 1}$$, where **P** refers to the system response matrix, **Y** refers to the acquired scanned PET projection data, $I^k$ includes the initial PET image of each axial slice in the k-th iteration, $P^T$ refers to the transposed matrix of the system response matrix, and **1** refers to an all-ones matrix.

Taking the axial slice$_{z\_i}$ as an example, after obtaining the image update information $I_{update}^k$ for the k-th iteration, the initial PET image $I_{z\_i\ i}^k$ of the axial slice$_{z\_i}$ in the k-th iteration can be updated based on the image update information $I_{update}^k$ for the k-th iteration to obtain the updated PET image $I'^k_{z\_i}$ of the axial slice$_{z\_i}$ in the k-th iteration.

Specifically, the image update information $I_{update}^k$ for the k-th iteration and the initial PET image $I_{z\_i}^k$ of the axial slice$_{z\_i}$ in the k-th iteration are multiplied, and the product result is used as the updated PET image $I'^k_{z\_i}$ of the axial slice$_{z\_i}$ in the k-th iteration.

Similarly, the initial PET images of other axial slices in the k-th iteration may be updated based on the image update information $I_{update}^k$ for the k-th iteration to obtain updated PET images of other axial slices in the k-th iteration.

In some embodiments, the method provided herein further includes:
obtaining second PET projection prediction data based on the weighted PET image of each axial slice, and determining whether the iteration end condition is met based on differences between the second PET projection prediction data and the PET projection data.

Based on the weighted PET image of each axial slice, PET projection prediction data can be obtained. This PET projection prediction data are obtained by prediction and, for purposes of distinction, are referred to as the second PET projection prediction data. On a condition that the weighted PET image of each axial slice is close to a true image, the second PET projection prediction data will be closer to acquired scanned PET projection data. Therefore, based on the difference between the second PET projection prediction data and the PET projection data, whether the iteration termination condition is met can be determined.

For example, on a condition that the current iteration is the first iteration, after obtaining the weighted image of each axial slice obtained in the first iteration, second PET projection prediction data can be generated based on the weighted image of each axial slice obtained in the first iteration. The difference between the second PET projection prediction data and the PET projection data obtained by the acquisition scan is obtained. On a condition that the difference is less than or equal to a difference threshold, it can be determined that the iteration end condition is met, and the weighted image of each axial slice obtained in the first iteration can be determined as the local medical image of each axial slice. On a condition that the difference is greater than the difference threshold, it can be determined that the iteration end condition is not met, and the weighted image of each axial slice obtained in the first iteration can be determined as the initial PET image of each axial slice in the second iteration, and the process proceeds to the second iteration.

Exemplarily, on a condition that the current iteration is not the first iteration, the current iteration is denoted as the k-th iteration. After obtaining the weighted image of each axial slice in the k-th iteration, second PET projection prediction data can be generated based on the weighted image of each axial slice in the k-th iteration. The difference between the second PET projection prediction data and the PET projection data acquired during the scan is obtained. On a condition that the difference is less than or equal to a difference threshold, it can be determined that the iteration end condition is met. In this case, the weighted image of each axial slice in the k-th iteration can be determined as the local medical image of each axial slice. On a condition that the difference is greater than the difference threshold, it can be determined that the iteration end condition is not met. In this case, the weighted image of each axial slice in the k-th iteration can be determined as the initial PET image of each axial slice in the k+1-th iteration, and the process proceeds to the k+1-th iteration.

A PET image reconstruction method is provided in the present disclosure, which includes the following steps.

PET projection data are acquired. At least one iterative reconstruction is performed on the PET projection data to obtain the local medical image of each axial slice. During a current iterative reconstruction, an initial PET image of each axial slice in a current iteration is updated based on the PET projection data to obtain an updated PET image of each axial slice in the current iteration. A weighted summation is performed on the updated PET image of each axial slice in the current iteration and the initial PET image of each axial slice in the current iteration using two weights obtained based on a relaxation factor of each axial slice, to obtain a weighted PET image of each axial slice in the current iteration. The closer the axial slice within a predetermined range is to a central axial slice, the greater the relaxation factor of the axial slice, the greater a weight assigned to the updated PET image of the axial slice in the current iteration, and the smaller a weight assigned to the initial PET image of the axial slice in the current iteration.

After performing a PET scan on a target object, a number of events detected by each LOR (LOR) is obtained. These events form the PET projection data, which are projection data acquired during the scan. For example, on a condition there are m LORs, the PET projection data include $\{y_{LOR\_1}, y_{LOR\_2}, ..., y_{LOR\_m}\}$, where $y_{LOR\_m}$ refers to the number of events detected by m-th LOR. The PET projection data can be expressed in a matrix format with m rows and one column, denoted by **Y**.

Different axial slices correspond to different axial positions. For example, two adjacent axial slices correspond to axial positions $z\_i$ and $z\_i+1$, respectively. These two axial slices can be denoted as $slice_{z\_i}$ and $slice_{z\_i+1}$. Among the axial slices, the axial slice that is aligned or nearly aligned with the axial position of a central detection ring is the central axial slice. The central detection ring can be a detection ring in the middle of a plurality of parallel detection rings.

In the present disclosure, corresponding relaxation factors for different axial slices are set, which can control the convergence degree of different axial slices and avoid image edge noise enhancement due to excessive convergence. Specifically, for any axial slice within the predetermined range, the closer the axial slice is to the central axial slice, the greater the relaxation factor of the axial slice. Accordingly, the weight assigned to the weighted PET image of the axial slice in each iteration is greater, and the weight assigned to the initial PET image of the axial slice in each iteration is smaller, thereby suppressing the image noise level in the axial edge region during the iterative reconstruction process.

Taking the axial $slice_{z\_i}$ as an example, the explanation is as follows.

(1) On a condition that the current iteration is the first iteration, the initial PET image (which can be denoted as $I^1_{z\_i}$) of the axial $slice_{z\_i}$ in the first iteration can be randomly generated. Based on the PET projection data, an updated PET image (which can be denoted as $I'^1_{z\_i}$) of the axial slice z_i in the first iteration is obtained.

Based on the relaxation factor of axial $slice_{z\_i}$, two weights can be obtained. Based on these two weights, a weighted summation is performed on the updated PET image $I'^1_{z\_i}$ of axial $slice_{z\_i}$ in the first iteration and the initial PET image $I^1_{z\_i}$ of axial $slice_{z\_i}$ in the first iteration to obtain the weighted PET image of axial $slice_{z\_i}$ in the first iteration. On a condition that axial $slice_{z\_i}$ is an axial slice within a predetermined range, the closer axial $slice_{z\_i}$ is to the central axial slice, the greater the relaxation factor of axial $slice_{z\_i}$, the greater the weight assigned to the updated PET image $I'^1_{z\_i}$ of axial $slice_{z\_i}$ in the first iteration, and the smaller the weight assigned to the initial PET image $I^1_{z\_i}$ of the axial slice in the first iteration.

In the above manner, PET images of other axial slices after weighting in the first iteration can also be obtained.

On a condition that the weighted PET image of each axial slice in the first iteration meets the iteration end condition, the weighted PET image of each axial slice in the first iteration can be determined as the local medical image of each axial slice.

On a condition that the weighted PET image of each axial slice in the first iteration do not meet the iteration end condition, the weighted PET image of each axial slice in the first iteration can be determined as the initial PET image of each axial slice in the next iteration, and the next iteration is performed based on the initial PET image of each axial slice in the next iteration.

(2) On a condition that the current iteration is not the first iteration, the current iteration is denoted as the k-th iteration, where k refers to a positive integer greater than or equal to 2. Then, the weighted PET image of the axial slice$_{z\_i}$ in the k-1-th iteration can be obtained, and the PET image is used as the initial PET image (which can be denoted as $I_{z\_i}^{k}$) of the axial slice$_{z\_i}$ in the k-th iteration. Based on the PET projection data, the updated PET image (which can be denoted as $I_{z\_i}'^{k}$) of the axial slice z_i in the k-th iteration is obtained.

Two weights can be obtained based on the relaxation factor of axial slice$_{z\_i}$. Based on these two weights, a weighted summation is performed on the updated PET image $I_{z\_i}'^{k}$ of axial slice$_{z\_i}$ in the k-th iteration and the initial PET image $I_{z\_i}^{k}$ of axial slice$_{z\_i}$ in the k-th iteration to obtain the weighted PET image of axial slice$_{z\_i}$ in the k-th iteration. On a condition that axial slice$_{z\_i}$ is an axial slice within a predetermined range, the closer axial slice$_{z\_i}$ is to the central axial slice, the greater the relaxation factor of axial slice$_{z\_i}$, the greater the weight assigned to the updated PET image $I_{z\_i}'^{k}$ of axial slice$_{z\_i}$ in the k-th iteration, and the smaller the weight assigned to the initial PET image $I_{z\_i}^{k}$ of axial slice$_{z\_i}$ in the k-th iteration.

In the above manner, weighted PET images of other axial slices in the k-th iteration can also be obtained.

On a condition that the weighted PET image of each axial slice in the k-th iteration meets the iteration end condition, the weighted PET image of each axial slice in the k-th iteration can be determined as the local medical image of each axial slice.

On a condition that the weighted PET image of each axial slice in the k-th iteration do not meet the iteration end condition, the weighted PET image of each axial slice in the k-th iteration can be determined as the initial PET image of each axial slice in the next iteration, and the next iteration is performed based on the initial PET image of each axial slice in the next iteration.

In the above-mentioned PET image reconstruction method, PET projection data are acquired. The PET projection data are iteratively reconstructed at least once to obtain a local medical image of each axial slice. During the current iterative reconstruction, the initial PET image of each axial slice in the current iteration is updated based on the PET projection data to obtain an updated PET image of each axial slice in the current iteration. A weighted summation is performed on two weights obtained based on the relaxation factor of each axial slice by taking the updated PET image of each axial slice in the current iteration and the initial PET image of each axial slice in the current iteration to obtain a weighted PET image of each axial slice in the current iteration. For any axial slice within a predetermined range, the closer the axial slice is to the central axial slice, the greater the relaxation factor of the axial slice, and accordingly, the greater the weight assigned to the weighted PET image of the axial slice in each iteration, and the smaller the weight assigned to the initial PET image of the axial slice in each iteration, thereby suppressing the image noise level in the axial edge region during the iterative reconstruction process.

It should be understood that, although the various steps in the flowcharts involved in the various embodiments described above are displayed in sequence according to the instructions of the arrows, these steps are not necessarily executed in sequence in the order indicated by the arrows. Unless otherwise specified herein, there is no strict order restriction on the execution of these steps, and these steps can be executed in other orders. Moreover, at least a portion of the steps in the flowcharts involved in the various embodiments described above can include a plurality of steps or a plurality of stages, and these steps or stages are not necessarily executed and completed at the same time, but can be executed at different times, and the execution order of these steps or stages is not necessarily to be carried out in sequence, but can be executed in turn or alternately with other steps or at least a portion of steps or stages in other steps.

Based on the same inventive concept, a PET image reconstruction apparatus, which is for implementing the aforementioned PET image reconstruction method, is further provided in embodiments of the present disclosure. The solution provided by this apparatus is similar to the solution described in the aforementioned method. Therefore, the specific limitations of one or more embodiments of the PET image reconstruction apparatus provided below can be found in the aforementioned limitations of the PET image reconstruction method and are not further elaborated here.

In some embodiments, as shown in FIG. 13, a PET image reconstruction apparatus is provided, including an updating module 501, a relaxation factor acquisition module 502, a weighted summation module 503, a target image acquisition module 504, and a return-to-execution module 505.

The updating module 501 is configured to update an initial PET image of each axial slice based on PET projection data to obtain an updated PET image of the axial slice.

The relaxation factor acquisition module 502 is configured to obtain a relaxation factor of each axial slice.

The weighted summation module 503 is configured to obtain, for each axial slice, a first weight and a second weight of the axial slice based on the relaxation factor of the axial slice, and perform a weighted summation on the updated PET image of the axial slice and the initial PET image of the axial slice based on the first weight and the second weight to obtain a weighted PET image of the axial slice.

The target image acquisition module 504 is configured to obtain the local medical image of each axial slice based on the weighted PET images of the axial slices on a condition that the weighted PET image of each axial slice meets an iteration end condition.

The return-to-execution module 505 is configured to determine the weighted PET image of each axial slice as the initial PET image of each axial slice on a condition that the weighted PET image of each axial slice does not meet the iteration end condition, and return to the updating module 501, the relaxation factor acquisition module 502 and the weighted summation module 503.

In some embodiments, the relaxation factor acquisition module 502 is further configured to obtain, for each axial slice, a slice number difference between the axial slice and a central axial slice, and determine, on a condition that the slice number difference is greater than a slice number difference threshold, the relaxation factor of the axial slice based on the slice number difference and a predetermined mapping relationship. the relaxation factor of the axial slice is smaller than a relaxation factor of the central axial slice.

In some embodiments, the relaxation factor acquisition module 502 is further configured to determine, on a condition that the slice number difference is less than or equal to the slice number difference threshold, the relaxation factor of the central axial slice as the relaxation factor of the axial slice.

In some embodiments, the relaxation factor acquisition module 502 is further configured to:

For each of the axial slices, a slice number difference between the axial slice and the central axial slice is obtained; and a relaxation factor of the axial slice is determined based on the slice number difference and a negative correlation function.

In some embodiments, the relaxation factor acquisition module 502 is further configured to obtain, for each voxel on the axial slice, a detection probability of each LOR for the voxel, obtain an attenuation coefficient of each LOR, and obtain, for each axial slice, the relaxation factor of the axial slice based on the detection probability of each LOR of each voxel on the axial slice and the attenuation coefficient of each LOR of each voxel.

In some embodiments, the relaxation factor acquisition module 502 is further configured to perform, for each voxel on the axial slice, a weighted summation on the detection probability of each LOR of the voxel and the attenuation coefficient of each LOR to obtain a detection probability weighted value of the voxel, and obtain the relaxation factor of the axial slice based on the detection probability weighted value of each voxel on the axial slice.

In some embodiments, the weighted summation module 503 is further configured to obtain the first weight of the axial slice based on the relaxation factor of the axial slice, and obtain the second weight of the axial slice based on a difference between a predetermined value and the relaxation factor of the axial slice.

In some embodiments, the updating module 501 is further configured to obtain first PET projection prediction data based on an initial PET image of each axial slice, and update the initial PET image of each axial slice based on differences between the first PET projection prediction data and the PET projection data to obtain the updated PET image of each axial slice.

In some embodiments, the apparatus further includes an iteration end judgment module configured to obtain second PET projection prediction data based on the weighted PET image of each axial slice, and determine whether the iteration end condition is met based on differences between the second PET projection prediction data and the PET projection data.

Each module in the aforementioned PET image reconstruction apparatus may be implemented in whole or in part via software, hardware, or a combination thereof. Each module may be embedded in or independent of a processor within a computer device in the form of hardware, or may be stored in a memory within the computer device in the form of software, so that the processor can call and execute the corresponding operations of each module.

Based on the same inventive concept, a PET image reconstruction apparatus, which is for implementing the aforementioned PET image reconstruction method, is further provided in embodiments of the present disclosure. The solution provided by this apparatus is similar to the solution described in the aforementioned method. Therefore, the specific limitations of one or more embodiments of the PET image reconstruction apparatus provided below can be found in the aforementioned limitations of the PET image reconstruction method and are not further elaborated here.

In some embodiments, a PET image reconstruction apparatus is provided, including a projection data acquisition module and an iterative reconstruction module.

The projection data acquisition module is configured to acquiring PET projection data.

The iterative reconstruction module is configured to perform at least one iterative reconstruction on the PET projection data to obtain the local medical image of each axial slice.

The iterative reconstruction module is further configured to update an initial PET image of each axial slice in a current iteration based on the PET projection data during a current iterative reconstruction, to obtain un updated PET image of each axial slice in the current iteration, and perform a weighted summation on the updated PET image of each axial slice in the current iteration and the initial PET image of each axial slice in the current iteration using two weights obtained based on a relaxation factor of each axial slice, to obtain a weighted PET image of each axial slice in the current iteration. The closer the axial slice within a predetermined range is to a central axial slice, the greater the relaxation factor of the axial slice, the greater a weight assigned to the updated PET image of the axial slice in the current iteration, and the smaller a weight assigned to the initial PET image of the axial slice in the current iteration.

Each module in the aforementioned PET image reconstruction apparatus may be implemented in whole or in part via software, hardware, or a combination thereof. Each module may be embedded in or independent of a processor within a computer device in the form of hardware, or may be stored in a memory within the computer device in the form of software, so that the processor can call and execute the corresponding operations of each module.

In an exemplary embodiment, a computer device is provided, the internal structure of which may be shown in FIG. 1. The computer device includes a processor, memory, an input/output (I/O) interface, and a communication interface. The processor, memory, and I/O interface are connected via a system bus, and the communication interface is connected to the system bus via the I/O interface. The processor of the computer device is configured to provide computing and control capabilities. The memory of the computer device includes a non-transitory storage medium and internal memory. The non-transitory storage medium stores an operating system, a computer program, and a database. The internal memory provides an environment for the operation of the operating system and computer program in the non-transitory storage medium. The database of the computer device is configured to store data related to the above-described method. The I/O interface of the computer device is configured to exchange information between the processor and an external device. The communication interface of the computer device is configured to communicate with an external terminal via a network connection. When executed by the processor, the computer program implements a PET image reconstruction method.

Those skilled in the art will understand that the structure shown in FIG. 1 is merely a block diagram of a portion of the structure related to the solution of the present disclosure, and does not constitute a limitation on the computer device to which the solution of the present disclosure is applied. The specific computer device may include more or fewer components than shown in the figure, or combine certain components, or have a different arrangement of components.

In some embodiments, a computer device is provided, including a memory and a processor. The memory stores a computer program, and the processor implements the steps in the above-mentioned various method embodiments when executing the computer program.

In some embodiments, a non-transitory computer-readable storage medium is provided, on which a computer program is stored. When the computer program is executed by a processor, the steps in the above-mentioned various method embodiments are implemented.

In some embodiments, a computer program product is provided, on which a computer program is stored, and the computer program is used by a processor to execute the steps in the above-mentioned various method embodiments.

It should be noted that the user information (including but not limited to user device information, user personal information, etc.) and data (including but not limited to data used for analysis, stored data, displayed data, etc.) involved in the present disclosure are all information and data authorized by the user or fully authorized by all parties, and the collection, use and processing of relevant data must comply with relevant regulations.

Those skilled in the art will appreciate that all or part of the processes in the above-mentioned embodiments can be implemented by instructing the relevant hardware through a computer program. The computer program can be stored in a non-transitory computer-readable storage medium. When the computer program is executed, it can include the processes of the above-mentioned embodiments. In particular, any reference to a memory, database, or other medium used in the embodiments provided in the present disclosure can include at least one of a non-transitory memory and a transitory memory. The non-transitory memory can include read-only memory (ROM), magnetic tape, floppy disk, flash memory, optical memory, high-density embedded non-transitory memory, resistive random access memory (ReRAM), magnetic random access memory (MRAM), ferroelectric random access memory (FRAM), phase change memory (PCM), graphene memory, etc. Transitory memory can include random access memory (RAM) or external cache memory, etc. By way of illustration and not limitation, RAM can take various forms, such as static random access memory (SRAM) or dynamic random access memory (DRAM). The databases involved in the various embodiments provided herein may include at least one of a relational database and a non-relational database. Non-relational databases may include, but are not limited to, blockchain-based distributed databases. The processors involved in the various embodiments provided herein may be, but are not limited to, general-purpose processors, central processing units (CPUs), graphics processing units (GPUs), digital signal processors (DSPs), programmable logic devices (PLDs), quantum computing-based data processing logic devices, artificial intelligence (AI) processors, and the like.

The technical features of the above embodiments can be combined arbitrarily. In order to make the description concise, not all possible combinations of the technical features in the above embodiments are described. However, as long as there is no contradiction in the combination of these technical features, they should be considered to be within the scope of the present disclosure.

The above-described embodiments merely represent several implementations of the present disclosure. While the descriptions are relatively specific and detailed, they should not be construed as limiting the scope of the present disclosure. It should be noted that a person of ordinary skill in the art may make various modifications and improvements without departing from the spirit of the present disclosure, and these modifications and improvements fall within the scope of protection of the present disclosure. Therefore, the scope of protection of the present disclosure shall be determined by the appended claims.

**Claims**

1.   An emission tomography image processing method, comprising:

  acquiring local medical images of a target object, wherein different local medical images correspond to different spatial positions or different tissue regions;
  predicting noise level information corresponding to the local medical images; and
  performing, based on the noise level information corresponding to the local medical images, image denoising on the local medical images to obtain a target medical image, wherein at least two of the local medical images correspond to different noise level information.

2.   The emission tomography image processing method according to claim 1, wherein the different spatial positions comprise different axial slices, optionally,
  wherein acquiring the local medical images of the target object comprises:

  acquiring the local medical images of the target object corresponding to different axial slices; and
  predicting the noise level information corresponding to the local medical images comprises:
  inputting the local medical images corresponding the axial slices into an axial slice noise prediction model, and predicting a noise level of the local medical images corresponding to the different axial slices using the axial slice noise prediction model to obtain noise level information corresponding to the different axial slices.

3.   The emission tomography image processing method according to any one of claims 1 to 2, wherein acquiring the local medical images of the target object comprises:

  acquiring local medical images of the target object corresponding to different tissue regions; and
  predicting the noise level information corresponding to the local medical images comprises:
  inputting the local medical images corresponding the tissue regions into a regional noise prediction model, and predicting a noise level of the local medical images corresponding to different tissue regions using the regional noise prediction model to obtain noise level information corresponding to the different tissue regions.

4.   The emission tomography image processing method according to any one of claims 1 to 3, wherein performing, based on the noise level information corresponding to the local medical images, image denoising on the local medical images to obtain the target medical image comprises:

  performing, based on the noise level information corresponding to the local medical images, image denoising on the local medical images using a predetermined denoising model to obtain the target medical image; optionally, wherein the method further comprises training the denoising model, and training the denoising model comprises:

  acquiring sample medical images and sample local medical images of a sample object, the sample local medical images comprising at least one of sample local medical images corresponding to a plurality of axial slices and sample local medical images corresponding to a plurality of tissue regions;
  predicting a noise level of the sample local medical images using a noise prediction model to obtain noise level information, the noise prediction model comprising at least one of an axial slice noise prediction model and a regional noise prediction model;
  fusing the noise level information with the sample medical images to obtain fused sample medical images; and
  training an initial denoising model using the fused sample medical images until an output result of the initial denoising model meets a predetermined condition, to obtain the denoising model.

5. The emission tomography image processing method according to claim 2, wherein the different axial slices have different relaxation factors; optionally,
wherein acquiring the local medical images of the target object corresponding to the different axial slices comprises:

in a step S1, updating an initial PET image of each axial slice based on PET projection data to obtain an updated PET image of the axial slice;
in a step S2, obtaining a relaxation factor of each axial slice;
in a step S3, obtaining, for each axial slice, a first weight and a second weight of the axial slice based on the relaxation factor of the axial slice, and performing a weighted summation on the updated PET image of the axial slice and the initial PET image of the axial slice based on the first weight and the second weight to obtain a weighted PET image of the axial slice;
in a step S4, obtaining, on a condition that the weighted PET image of each axial slice meets an iteration end condition, the local medical images corresponding to the axial slices based on the weighted PET images of the axial slices; and
in a step S5, determining, on a condition that the weighted PET image of each axial slice does not meet the iteration end condition, the weighted PET image of each axial slice as the initial PET image of each axial slice, and returning to execute steps S1 to S3.

6. The emission tomography image processing method according to claim 5, wherein obtaining the relaxation factors of the axial slices comprises:

obtaining, for each axial slice, a slice number difference between the axial slice and a central axial slice; and
on a condition that the slice number difference is greater than a slice number difference threshold, determining the relaxation factor of the axial slice based on the slice number difference and a predetermined mapping relationship, wherein the relaxation factor of the axial slice is smaller than a relaxation factor of the central axial slice, optionally,
wherein the method further comprises:
on a condition that the slice number difference is less than or equal to the slice number difference threshold, determining the relaxation factor of the central axial slice as the relaxation factor of the axial slice.

7. The emission tomography image processing method according to claim 5, wherein obtaining the relaxation factors of the axial slices comprises:

obtaining, for each voxel on the axial slice, a detection probability of each line of response (LOR) for the voxel;
obtaining an attenuation coefficient of each LOR; and
obtaining, for each axial slice, the relaxation factor of the axial slice based on the detection probability of each LOR of each voxel on the axial slice and the attenuation coefficient of each LOR of each voxel, optionally,
wherein obtaining, for each axial slice, the relaxation factor of the axial slice based on the detection probability of each LOR of each voxel on the axial slice and the attenuation coefficient of each LOR of each voxel comprises:

performing, for each voxel on the axial slice, a weighted summation on the detection probability of each LOR of the voxel and the attenuation coefficient of each LOR to obtain a detection probability weighted value of the voxel; and
obtaining the relaxation factor of the axial slice based on the detection probability weighted value of each voxel on the axial slice.

8. The emission tomography image processing method according to any one of claims 6 to 7, wherein obtaining the first weight and the second weight of the axial slice based on the relaxation factor of the axial slice comprises:

obtaining the first weight of the axial slice based on the relaxation factor of the axial slice; and
obtaining the second weight of the axial slice based on a difference between a predetermined value and the relaxation factor of the axial slice.

9. The emission tomography image processing method according to claim 5, wherein updating the initial PET image of each axial slice based on the PET projection data to obtain the updated PET image of each axial slice comprises:

obtaining first PET projection prediction data based on an initial PET image of each axial slice; and
updating the initial PET image of each axial slice based on differences between the first PET projection prediction data and the PET projection data to obtain the updated PET image of each axial slice.

10. The emission tomography image processing method according to claim 5, wherein the method further comprises:

obtaining second PET projection prediction data based on the weighted PET image of each axial slice; and determining whether the iteration end condition is met based on differences between the second PET projection prediction data and the PET projection data.

11. The emission tomography image processing method according to claim 10, wherein the iteration end condition comprises that a mean square error or a sum of absolute differences between the second PET projection prediction data and the PET projection data is less than a difference threshold.

12. The emission tomography image processing method according to claim 4, wherein the noise level information is scaled or smoothed before fusing with the sample medical images.

13. An emission tomography image processing apparatus, comprising:

an acquisition module configured to acquire local medical images of a target object, wherein different local medical images correspond to different spatial positions or different tissue regions; a prediction module configured to predict noise level information corresponding to the local medical images; and a denoising module configured to perform, based on the noise level information corresponding to the local medical images, image denoising on the local medical images to obtain a target medical image, wherein at least two of the local medical images correspond to different noise level information.

14. A computer device comprising a memory and a processor, the memory storing a computer program, wherein the processor, when executing the computer program, implements steps of the emission tomography image processing method according to any one of claims 1 to 11.

15. A non-transitory computer-readable storage medium having a computer program stored thereon, wherein a processor, when executing the computer program, implements steps of the emission tomography image processing method according to any one of claims 1 to 11.

FIG. 1

Acquire local medical images of the target object, where different local medical images correspond to different spatial positions or different tissue regions ⸺ S201

Predict noise level information corresponding to the local medical images ⸺ S202

Perform image denoising on the local medical image based on the noise level information corresponding to the local medical image to obtain a target medical image, where at least two of the local medical images correspond to different noise level information ⸺ S203

FIG. 2

FIG. 3

Acquire local medical images corresponding to different axial slices ⎯S301

input the local medical images corresponding to the axial slices into an axial slice noise prediction model, and predict a noise level of the local medical images corresponding to different axial slices using the axial slice noise prediction model to obtain noise level information corresponding to the different axial slices ⎯S302

Acquire local medical images corresponding to different tissue regions ⎯S303

Input the local medical images of the tissue regions into a regional noise prediction model, and predict a noise level of the local medical images corresponding to different tissue regions using the regional noise prediction model to obtain noise level information corresponding to the different tissue regions ⎯S304

input the local medical images corresponding to different axial slices and corresponding noise level information, the local medical images corresponding to different tissue regions and corresponding noise level information into a denoising model, and perform image denoising on the local medical images using the denoising model to obtain the target medical image ⎯S305

FIG. 4

Acquire sample medical images and sample local medical images of a sample object, the sample local medical image includes at least one of sample local medical images corresponding to a plurality of axial slices and sample local medical images corresponding to a plurality of tissue regions ⎯S401

Predict a noise level of the sample local medical images using a noise prediction model to obtain noise level information, the noise prediction model includes at least one of an axial slice noise prediction model and a regional noise prediction model ⎯S402

Fuse the noise level information with the sample medical images to obtain fused sample medical images ⎯S403

Train an initial denoising model using the fused sample medical images until an output result of the initial denoising model meets a predetermined condition, to obtain the denoising model ⎯S404

FIG. 5

FIG. 6

Local medical images corresponding to different axial slices → Axial Slice Noise Prediction Model

Local medical images corresponding to different tissue regions → Regional Noise Prediction Model

→ Denoising Model

FIG. 7

Emission Tomography Image Processing Apparatus

Acquisition Module — 11

Prediction Module — 12

Denoising Module — 13

FIG. 8

Ring_1
Ring_2
Ring_3
Ring_4

y
z
x

FIG. 9

| | |
|---|---|
| Update an initial PET image of each axial slice based on PET projection data to obtain an updated PET image of the axial slice | S1 |
| Obtain a relaxation factor of each axial slice | S2 |
| for each axial slice, obtain a first weight and a second weight of the axial slice based on the relaxation factor of the axial slice, and perform a weighted summation on the updated PET image of the axial slice and the initial PET image of the axial slice based on the first weight and the second weight, to obtain a weighted PET image of the axial slice | S3 |
| On a condition that the weighted PET image of each axial slice meets an iteration end condition, obtain the local medical images of the axial slices based on weighted PET images of the axial slices | S4 |
| On a condition that the weighted PET image of each axial slice does not meet the iteration end condition, determine the weighted PET image of each axial slice as the initial PET image of each axial slice, and steps S1 to S3 are to be returned to for execution | S5 |

FIG. 10

FIG. 11 (a)

FIG. 11 (b)

FIG. 12

FIG. 13

**EUROPEAN SEARCH REPORT**

Application Number

EP 25 21 1092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/051674 A1 (GOOSSENS BART [BE] ET AL) 28 February 2013 (2013-02-28) | 1,3,4, 12-15 | INV. G06T5/60 |
| Y | * abstract * | 2,5 | G06T5/70 |
| A | * paragraph [0001] * <br> * paragraph [0061] * <br> * paragraph [0088] - paragraph [0090] * <br> * figure 1 * <br> ----- | 6-11 | G06T12/10 |
| Y | EP 3 576 049 A2 (CANON MEDICAL SYSTEMS CORP [JP]) 4 December 2019 (2019-12-04) <br> * abstract * <br> * paragraph [0041] - paragraph [0048] * <br> * figure 4 * <br> ----- | 2,5 | |
| A | EP 4 261 775 A1 (KONINKLIJKE PHILIPS NV [NL]) 18 October 2023 (2023-10-18) <br> * the whole document * <br> ----- | 1-15 | |

TECHNICAL FIELDS SEARCHED (IPC)

G06T

The present search report has been drawn up for all claims

| Place of search | Date of completion of the search | Examiner |
|---|---|---|
| Munich | 4 March 2026 | Rusu, Alexandru |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 1092

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2026

| Patent document cited in search report | | Publication date | Patent family member(s) | | Publication date |
|---|---|---|---|---|---|
| US 2013051674 | A1 | 28-02-2013 | EP | 2385494 A1 | 09-11-2011 |
| | | | EP | 2567357 A1 | 13-03-2013 |
| | | | US | 2013051674 A1 | 28-02-2013 |
| | | | WO | 2011138044 A1 | 10-11-2011 |
| EP 3576049 | A2 | 04-12-2019 | EP | 3576049 A2 | 04-12-2019 |
| | | | EP | 4181057 A1 | 17-05-2023 |
| | | | JP | 7381224 B2 | 15-11-2023 |
| | | | JP | 7586999 B2 | 19-11-2024 |
| | | | JP | 2019211475 A | 12-12-2019 |
| | | | JP | 2024016161 A | 06-02-2024 |
| | | | US | 2019365341 A1 | 05-12-2019 |
| | | | US | 2022104787 A1 | 07-04-2022 |
| | | | US | 2022110600 A1 | 14-04-2022 |
| | | | US | 2024008832 A1 | 11-01-2024 |
| EP 4261775 | A1 | 18-10-2023 | CN | 119072714 A | 03-12-2024 |
| | | | EP | 4261775 A1 | 18-10-2023 |
| | | | EP | 4508595 A1 | 19-02-2025 |
| | | | JP | 2025513681 A | 30-04-2025 |
| | | | US | 2025252560 A1 | 07-08-2025 |
| | | | WO | 2023198645 A1 | 19-10-2023 |

EPO FORM P0459